# FASCICULE DE BREVET EUROPEEN

(11) **EP 2 986 728 B1**
(45) Date de publication et mention de la délivrance du brevet: **01.05.2019**
(21) Numéro de dépôt: 14722279.8
(22) Date de dépôt: 15.04.2014
(51) Int. Cl.: C12N 15/82, C07K 14/435

(54) **PRODUCTION COMMERCIALE DE PEPTIDASES C1A PAR EXPRESSION TRANSITOIRE CHEZ LES PLANTES**
KOMMERZIELLE HERSTELLUNG VON PEPTIDASEN C1A MITTELS TRANSIENTER EXPRESSION IN PFLANZEN
COMMERCIAL PRODUCTION OF PEPTIDASES C1A BY TRANSIENT EXPRESSION IN PLANTS

(30) Priorité: 15.04.2013 FR 1353386
(43) Date de publication de la demande: 24.02.2016
(73) Titulaire: Angany Inc., Québec, QC G1R 1R2 (CA)
(72) Inventeur: GOMORD, Véronique, F-76000 Rouen (FR); FITCHETTE, Anne-Catherine, F-76570 Pavilly (FR); CATALA, Virginie, F-27950 Saint Marcel (FR); FAYE, Loïc, F-76160 St Jacques Sur Darnetal (FR)
(74) Mandataire: Cabinet Plasseraud
(86) Numéro de dépôt international: PCT/FR2014/050916
(87) Numéro de publication internationale: WO 2014/170597

(56) Documents cités:
- EP-A1- 1 908 776
- FR-A1- 2 841 906
- DAVID LIENARD ET AL: "Suspension-cultured BY-2 tobacco cells produce and mature immunologically active house dust mite allergens", PLANT BIOTECHNOLOGY JOURNAL, vol. 5, no. 1, 1 janvier 2007 (2007-01-01) , pages 93-108, XP055037826, ISSN: 1467-7644, DOI: 10.1111/j.1467-7652.2006.00221.x
- GOMORD V ET AL: "Plante et medicament, un nouveau depart", BIOFUTUR, LAVOISIER, CACHAN, FR, vol. 1996, no. 154, 1 avril 1996 (1996-04-01), pages 26-30, XP004296772, ISSN: 0294-3506, DOI: 10.1016/S0294-3506(96)80021-4
- GOMORD V ET AL: "Protein retention and localization in the endoplasmic reticulum and the Golgi apparatus", BIOCHIMIE, MASSON, PARIS, FR, vol. 81, no. 6, 1 juin 1999 (1999-06-01), pages 607-618, XP027141335, ISSN: 0300-9084 [extrait le 1999-06-01]
- STEPHEN J. STREATFIELD: "Approaches to achieve high-level heterologous protein production in plants", PLANT BIOTECHNOLOGY JOURNAL, vol. 5, no. 1, 1 janvier 2007 (2007-01-01) , pages 2-15, XP055043727, ISSN: 1467-7644, DOI: 10.1111/j.1467-7652.2006.00216.x
- SAINT-JORE-DUPAS ET AL: "From planta to pharma with glycosylation in the toolbox", TRENDS IN BIOTECHNOLOGY, ELSEVIER PUBLICATIONS, CAMBRIDGE, GB, vol. 25, no. 7, 1 juillet 2007 (2007-07-01), pages 317-323, XP022116342, ISSN: 0167-7799
- GOMORD V ET AL: "Biopharmaceutical production in plants: problems, solutions and opportunities", TRENDS IN BIOTECHNOLOGY, ELSEVIER PUBLICATIONS, CAMBRIDGE, GB, vol. 23, no. 11, 1 novembre 2005 (2005-11-01), pages 559-565, XP027778149, ISSN: 0167-7799 [extrait le 2005-11-01]
- FAYE L ET AL: "Protein modifications in the plant secretory pathway: current status and practical implications in molecular pharming", VACCINE, ELSEVIER LTD, GB, vol. 23, no. 15, 7 mars 2005 (2005-03-07), pages 1770-1778, XP027652238, ISSN: 0264-410X [extrait le 2005-03-07]
- LEROUGE P ET AL: "N-GLYCOSYLATION OF RECOMBINANT PHARMACEUTICAL GLYCOPROTEINS PRODUCED IN TRANSGENIC PLANTS: TOWARDS AN HUMANISATION OF PLANT N-GLYCANS", CURRENT PHARMACEUTICAL BIOTECHNOLOGY, BENTHAM SCIENCE PUBLISHERS, NL, vol. 1, no. 2, 1 janvier 2000 (2000-01-01) , pages 347-354, XP001010224, ISSN: 1389-2010, DOI: 10.2174/1389201003378843
- LOÏC FAYE ET AL: "Success stories in molecular farming-a brief overview", PLANT BIOTECHNOLOGY JOURNAL, vol. 8, no. 5, 9 mai 2010 (2010-05-09), pages 525-528, XP055038398, ISSN: 1467-7644, DOI: 10.1111/j.1467-7652.2010.00521.x
- "Des solutions innovantes pour le traitement des allergies", , 9 avril 2010 (2010-04-09), XP055127607, Extrait de l'Internet: URL:http://www.cnrs.fr/dire/creation-entre prises/docs/2010I_ANGANY GENETICS.pdf [extrait le 2014-07-08]
- GIANPIERO MARCONI ET AL: "expression of a mature Der p 1 allergen isolated from an Italian strain of", TRANSGENIC RESEARCH, KLUWER ACADEMIC PUBLISHERS-PLENUM PUBLISHERS, NE, vol. 21, no. 3, 9 septembre 2011 (2011-09-09), pages 523-535, XP035052967, ISSN: 1573-9368, DOI: 10.1007/S11248-011-9551-5
- KREBITZ M ET AL: "RAPID PRODUCTION OF THE MAJOR BIRCH POLLEN ALLERGEN BET V 1 IN NICOTIANA BENTHAMIANA PLANTS AND ITS IMMUNOLOGICAL IN VITRO AND IN VIVO CHARACTERIZATION", FASEB JOURNAL, FED. OF AMERICAN SOC. FOR EXPERIMENTAL BIOLOGY, US, vol. 14, no. 10, 1 juillet 2000 (2000-07-01), pages 1279-1288, XP001148445, ISSN: 0892-6638, DOI: 10.1096/FJ.14.10.1279

## Description

La présente invention se rapporte à une cellule végétale comprenant une molécule d'ADN comprenant au moins une séquence nucléotidique hétérologue codant pour une préproprotéine d'une peptidase C1A liée de manière fonctionnelle à un promoteur fort, afin d'exprimer par expression transitoire une peptidase C1A mature et active dans la plante.

Les protéases ont une place prépondérante sur le marché mondial des enzymes, estimé à environ US$ 3 milliards (Leary et al., 2009, Mar Policy 33, 183-194), car elles jouent un rôle important dans la biotechnologie. En effet, d'une part, les protéases interviennent en tant que molécules d'intérêt, en tant que telles ; d'autre part elles permettent de réaliser des clivages protéolytiques qui modifient les propriétés chimiques, physiques, biologiques et immunologiques des protéines. Ainsi, les protéases sont des enzymes parmi les plus importantes, en raison de leurs multiples applications dans les industries alimentaires (brasserie, viandes, produits laitiers, présure), pharmaceutiques et l'industrie des détergents (Doran, 2002, Plant biotechnology and transgenic plants. New York; Mercel Dekker inc,; 143-163).

Parmi les protéases les plus couramment utilisées, on trouve les peptidases de la famille C1A. Les peptidases C1A (ou protéases C1A) sont des protéases à cystéine (CysProt), également connues sous le nom de thiol protéases, largement distribuées parmi les organismes vivants. Ainsi on les retrouve chez les procaryotes et les eucaryotes (par exemple les bactéries, les parasites, les plantes, les invertébrés et les vertébrés). Le mécanisme catalytique de ces enzymes implique une cystéine dans le site actif.
La papaïne, enzyme végétale, est le membre le plus étudié parmi les protéases à cystéine. Un grand nombre de CysProt ont été isolées à partir des plantes, comme l'actinidine, la protéinase Ω, la chymopapaïne et la bromélaïne. Les CysProt les plus utilisées sont les enzymes lysosomales telles que les caspases, les enzymes vacuolaires telle que la papaïne, l'actinidine et la bromelaine, et enfin les protéases de type cathepsines (Palma et al., 2002, Plant Physiol. biochem, 40: 521-530).
Cette famille de protéases est une cible potentielle de médicaments pharmaceutiques pour traiter des maladies caractérisées par une dégradation excessive de la matrice extracellulaire telles que l'ostéoporose, l'arthrite, les maladies vasculaires et le cancer. Elles ont également été identifiées comme des acteurs essentiels de la croissance, de la différenciation cellulaire, de la signalisation, et de l'invasion de l'hôte par divers agents pathogènes de l'homme, notamment des parasites pathogènes causant le paludisme, la maladie de Chagas et la schistosomiase. Enfin, les peptidases de la famille C1A constituent également le groupe 1 des allergènes et sont reconnues comme des allergènes majeurs. A ce titre, elles sont actuellement une cible pour le traitement des allergies par désensibilisation (Lecaille et al., 2002, Chem Rev, 102: 4459-4488 et McKerrow et al., 2006, Annu Rev Pathol, 1: 497-536).

Les CysProt partagent des caractéristiques communes. Ainsi, toutes les protéases C1A présentent des ponts disulfures nécessaires pour leur structure fonctionnelle ; elles contiennent une triade catalytique formée par trois résidus d'acides aminés conservés (Cys, His et Asn), et un résidu Gln impliqué dans le maintien de la conformation de l'enzyme sous forme active. Les CysProt sont synthétisées sous forme de précurseurs inactifs ou peu actifs (préproprotéines) pour empêcher la protéolyse inappropriée. La préproprotéine comprend un peptide signal, un propeptide N-terminal de 130 à 150 acides aminés, et la protéine mature d'environ 220 à 270 acides aminés. L'activation de ces protéases a lieu soit sous l'action d'enzymes de maturation, soit par autocatalyse. L'activation a lieu par protéolyse intra- ou intermoléculaire contrôlée, en particulier par clivage du propeptide inhibiteur (Wiederanders et al. 2003, Current Protein and Peptide Science, 4, 309-326). Ce propeptide joue ainsi un rôle important en tant que modulateur de l'activité de la protéase, afin de garantir que la protéase soit maturée au bon endroit et/ou au bon moment (Demidyuk et al. 2010, J. Biol. Chem, 285, 2003-2013).

À ce jour, de nombreux efforts ont été réalisés pour exprimer les CysProt sous forme recombinante dans une grande variété de systèmes d'expression (Brômme et al., 2004, Methods, 32, 199-206). Ces travaux ont très souvent conduit à des échecs compte tenu de la structure complexe des peptidases de la famille C1A.

Il existe donc un besoin pour produire les CysProt, et notamment les peptidases C1A, sous forme recombinante, et ce de façon reproductible et efficace (avec un bon rendement). Il existe également un besoin pour produire des peptidases C1A à des niveaux commercialement et économiquement acceptables.

De façon surprenante étant donné leur toxicité *in vivo,* les inventeurs ont maintenant découvert que la production de peptidases C1A sous forme recombinante est possible, par expression transitoire chez les plantes. Cette production, qui se fait dans une cellule végétale, permet l'obtention d'une peptidase C1A sous forme mature et active, avec un bon rendement, de façon très simple et reproductible. En outre, la peptidase C1A obtenue n'est pas contaminée par des protéases et/ou d'autres contaminants d'origine animale. Les inventeurs ont ainsi pu obtenir une production, à des niveaux commercialement acceptables, de plusieurs peptidases C1A.

L'invention a donc pour objet une cellule végétale comprenant une molécule d'ADN comprenant au moins une séquence nucléotidique hétérologue codant pour une préproprotéine d'une peptidase C1A liée de manière fonctionnelle à un promoteur fort, de préférence un promoteur 35S. La molécule d'ADN liée au promoteur fort permet d'exprimer de façon transitoire une peptidase C1A mature et active dans la cellule. De préférence, ladite cellule végétale est une cellule de *Nicotiana benthamiana.* De préférence, la cellule végétale selon l'invention comprend une molécule d'ADN comprenant au moins une séquence nucléotidique hétérologue codant pour une préproprotéine d'une peptidase C1A liée de manière fonctionnelle à un promoteur fort, de préférence un promoteur 35S, ladite molécule d'ADN n'étant pas intégrée dans le génome de la cellule végétale.
L'invention a également pour objet une plante comprenant au moins une cellule végétale selon l'invention.
Un autre objet de l'invention est de fournir un procédé de production d'une peptidase C1A, comprenant l'expression de ladite peptidase C1A dans une cellule végétale selon l'invention, ou dans une plante comprenant une telle cellule.
Un autre objet de l'invention se rapporte à une peptidase C1A susceptible d'être obtenue par le procédé selon l'invention, avec une cellule végétale selon l'invention, ou avec une plante selon l'invention. La peptidase ainsi obtenue peut être utilisée comme médicament. Elle peut également être utilisée dans le diagnostic d'allergies, et être intégrée dans un kit de diagnostic d'allergies.

La cellule végétale selon l'invention comprend une molécule d'ADN liée de manière fonctionnelle à un promoteur fort, de préférence un promoteur 35S. Par « lié de manière fonctionnelle », on entend que la molécule d'ADN est fusionnée avec le promoteur fort, de telle sorte que le promoteur fort induise la transcription de la molécule d'ADN.
Cela permet l'expression de ladite molécule d'ADN sous forme transitoire, i.e. sans intégration de l'ADN, notamment l'ADNc, dans le génome de la cellule végétale. En effet, l'utilisation de l'expression transitoire dans une cellule végétale ou une plante selon l'invention permet d'augmenter les rendements de production des protéases C1A sous forme active jusqu'à des niveaux élevés, compatibles avec une exploitation commerciale, mais incompatibles avec la survie d'une plante qui exprimerait ces protéases toxiques de façon stable. Cette toxicité pour l'hôte d'expression est notamment illustrée par l'impossibilité de produire la protéase Der p1 sous forme active chez la levure P. pastoris ou chez la bactérie E. coli. Dans le cas d'une expression transitoire, la récolte de la biomasse végétale a lieu lors du pic d'expression de la protéine recombinante, i.e. typiquement 4 à 6 jours après la transfection.
De préférence, la cellule végétale selon l'invention comprend un vecteur d'expression comprenant au moins une séquence nucléotidique hétérologue codant pour une préproprotéine d'une peptidase C1A liée de manière fonctionnelle à un promoteur fort, de préférence un promoteur 35S.

Dans un mode de réalisation préféré, la peptidase C1A est choisie parmi les peptidases C1A d'acariens et les peptidases C1A végétales. De préférence, la peptidase C1A d'origine végétale est une peptidase C1A d'Asteraceae ou de Fabaceae.
Les gènes codant pour les peptidases C1A sont bien connus. Ainsi, un grand nombre de ces gènes ont été clonés et caractérisés (http://merops.sanger.ac.uk/cgi-bin/famsum?family=C1). A titre d'exemple, parmi les quelque 800 protéases codées par les génomes de plantes, plus de 140 correspondent à des cystéine-protéases (CysProt) qui peuvent être regroupés en 15 familles et en 5 clans (Rawlings et al. 2010, Nucleic acids Res, 38, D227-233), dont les papaïne-protéases C1A (clan CA, famille C1, sous-famille C1A), encore subdivisés en protéines de type cathepsine L, B, H ou F en fonction de leurs structures génétiques et des relations phylogénétiques (Martinez and Diaz, 2008, BMC Evol Biol, 8, 198-209).
Plus préférentiellement, la peptidase C1A est choisie parmi Der p1 (peptidases C1A du groupe 1 de Dermatophagoides pteronyssinus), Der f1 (peptidases C1A du groupe 1 de Dermatophagoides farinae), Eur m1 (peptidases C1A du groupe 1 de Euroglyphus maynei), PEPC1 (Cysteine peptidase - CysP d'*Aster tripolium*), SH-EP (Sulfhydryl-endopeptidase-vignain de *Vigna mungo*), AMB (peptidases C1A d'*Ambrosia artemisiifolia,* d'*Ambrosia psilostachya* et d'*Ambrosia trifida*) et les protéines présentant au moins 70%, de préférence au moins 80%, de préférence au moins 90%, de préférence au moins 95%, de préférence au moins 99% d'identité avec l'une de ces dernières. Plus préférentiellement, la peptidase C1A est choisie parmi Der p1 (peptidases C1A du groupe 1 de Dermatophagoides pteronyssinus), Der f1 (peptidases C1A du groupe 1 de Dermatophagoides farinae), Eur m1 (peptidases C1A du groupe 1 de Euroglyphus maynei), PEPC1 (Cysteine peptidase - CysP d'*Aster tripolium*), SH-EP (Sulfhydryl-endopeptidase-vignain de *Vigna mungo*), AMB (peptidases C1A d'*Ambrosia artemisiifolia,* d'*Ambrosia psilostachya* et d'*Ambrosia trifida*) et de préférence AMB A11 (qui correspond à la séquence nucléique KF528831 dans GenBank, ayant 1161 paires de bases, ou à la séquence protéique AHA56102.1 dans GenBank, ayant 386 acides aminés), la séquence SEQ ID NO : 10, la séquence SEQ ID NO : 11, la séquence SEQ ID NO : 12 et les protéines présentant au moins 70%, de préférence au moins 80%, de préférence au moins 90%, de préférence au moins 95%, de préférence au moins 99% d'identité avec l'une de ces dernières.

Par "pourcentage d'identité" entre deux séquences d'acides aminés au sens de la présente invention, on entend désigner un pourcentage de résidus d'acides aminés identiques entre les deux séquences à comparer, obtenu après le meilleur alignement, ce pourcentage étant purement statistique et les différences entre les deux séquences étant réparties au hasard et sur toute leur longueur. Par "meilleur alignement" ou "alignement optimal", on entend l'alignement pour lequel le pourcentage d'identité déterminé comme ci-après est le plus élevé. Les comparaisons de séquences entre deux séquences d'acides aminés sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (1981, J. Mol Evol., 18:38-46), au moyen de l'algorithme d'homologie locale de Neddleman et Wunsch (1970), au moyen de la méthode de recherche de similarité de Pearson et Lipman (1988, PNAS, 85: 2444-2448), au moyen de logiciels informatiques utilisant ces algorithmes (GAP, BESTFIT, BLAST P, BLAST N, FASTA et TFASTA dans le Wisconsin Genetics Software Package, Genetics Computer Group, 575 Science Dr., Madison, WI).

La molécule d'ADN comprend au moins une séquence nucléotidique hétérologue codant pour une préproprotéine de la peptidase C1A. De préférence, les préproprotéines présentant au moins 70%, de préférence au moins 80%, de préférence au moins 90%, de préférence au moins 95%, de préférence au moins 99% d'identité avec des préproprotéines sauvages sont également d'intérêt. De préférence, la préproprotéine est choisie parmi la séquence SEQ ID NO : 14, la séquence SEQ ID NO : 15 et la séquence SEQ ID NO : 16.
La préproprotéine de la peptidase C1A comprend un peptide signal, un propeptide et la protéine mature C1A. La séquence nucléotidique hétérologue codant la préproprotéine selon l'invention comprend donc une séquence codant le peptide signal, une séquence codant le propeptide et une séquence codant la protéine mature C1A.
Le peptide signal est notamment tout peptide signal reconnu par la cellule végétale, provenant ou non d'une peptidase C1A. Il cible notamment la peptidase C1A dans le milieu intercellulaire. De préférence, le peptide signal est celui de la chitinase de tabac, ou le peptide signal naturel de la peptidase.
Le propeptide est, quant à lui, issu d'une peptidase C1A. Le propeptide contient le motif GxNxFxD qui semble être essentiel pour un clivage correct de la forme précurseur de la protéase. La signature non contiguë ERFNIN (Ex3Rx3Fx3Nx3I/Vx3N) est spécifique des cathepsines L et H-like, alors que l'on observe une signature ERFNAQ chez la cathepsine F-like. Cette signature est de fonction inconnue (Grudkowska et Zagdanska 2004, Acta Biochim Pol, 51:609-624 Martinez and Diaz, 2008,BMC Evol Biol, 8, 198-209). En revanche, les protéases de type cathepsine B ne portent pas cette signature (Wiederanders 2003, Current Protein and Peptide Science, 4, 309-326; Martinez Diaz et 2008, BMC Evol Biol, 8, 198-209). En outre, certaines cathepsines L peuvent également contenir une extension C-terminale, qui comprend une région riche en Pro et un domaine granuline présentant une forte homologie avec les protéases de la famille épithéline/granuline des animaux (Yamada et al. 2001, Plant Physiol, 127: 1626-1634).
Dans un mode de réalisation préféré, la séquence nucléotidique hétérologue selon l'invention comprend, comme propeptide, un propeptide de peptidases C1A, notamment d'acariens ou d'origine végétale. De préférence, le propeptide de la préproprotéine de la peptidase C1A est le propeptide naturel ou le propeptide muté ou non de Der p1. De préférence, le propeptide de la préproprotéine de la peptidase C1A est choisi parmi le propeptide naturel de la peptidase C1A, le propeptide naturel de SH-EP (séquence SEQ ID NO : 13), la séquence SEQ ID NO :8 (i.e. les acides aminés 19 à 98 de la séquence Uniprot p08176), la séquence SEQ ID NO :9 (i.e. SEQ ID NO :8 modifiée par substitution de la glutamine en position 80 par une lysine) et la séquence SEQ ID NO :8 ou 9 mutée sur l'asparagine 16.

De préférence, la séquence de la protéine mature C1A est choisie parmi les séquences matures de Der p1, les séquences matures de Der f1, les séquences matures de Eur m1, la séquence mature de PEPC1, la séquence mature de SH-EP, la séquence SEQ ID NO : 10, la séquence SEQ ID NO : 11, la séquence SEQ ID NO : 12 et les protéines présentant au moins 70%, de préférence au moins 80%, de préférence au moins 90%, de préférence au moins 95%, de préférence au moins 99% d'identité avec l'une de ces dernières.

Selon l'invention, la séquence nucléotidique hétérologue codant la préproprotéine peut être obtenue à partir d'un gène connu déjà cloné codant pour une peptidase C1A, ou bien par criblage d'une banque d'ADNc avec des anticorps anti-protéase. Les méthodes utilisées sont bien connues de l'homme de l'art, et incluent notamment l'identification du gène par hybridation avec des sondes, PCR, séquençage et clonage moléculaire. Il est également possible de synthétiser le gène pour refléter l'utilisation des codons préférés chez les plantes ; dans ce cas, on parle d'optimisation de codons, souvent utile pour une forte expression des protéases sélectionnées (Murray et al, Nucleic Acid Res. 17:477 498 (1980)).

Dans un mode de réalisation préféré de l'invention, la séquence nucléotidique hétérologue codant la préproprotéine est liée à une séquence (appelée séquence d'adressage) permettant de cibler un compartiment de stockage de la peptidase C1A, afin de contrôler sa maturation. Cette maturation est nécessaire pour l'obtention des protéases sous forme active. Elle comprend en particulier le clivage protéolytique du propeptide N-terminal ; ce dernier a lieu lorsque la préproprotéine de la peptidase C1A est exprimée chez les plantes. Au contraire, il n'est pas effectué dans les systèmes de production bactériens ou de levures. Dans un mode de réalisation préférée de la présente invention, la peptidase C1A est produite en fusion avec son propre propeptide ou en fusion avec le propeptide de la peptidase C1A d'acariens ou en fusion avec un propeptide de peptidase C1A végétale, afin d'augmenter les rendements de production de la protéine.

De préférence, la séquence nucléotidique hétérologue codant la préproprotéine selon l'invention comprend en outre une séquence peptidique d'adressage intracellulaire. De préférence, cette séquence peptidique d'adressage cible la peptidase C1A sous forme soluble ou membranaire vers le réticulum endoplasmique ou les différents compartiments constitutifs du système endomembranaire de sécrétion de la cellule végétale.

De préférence, la séquence nucléotidique hétérologue codant la préproprotéine selon l'invention est mutée afin de contrôler la maturation de la protéase. Dans ce cas, elle peut être mutée de manière à bloquer le clivage protéolytique, par exemple en utilisant une version modifiée du propeptide de Der p1, le propeptide-Lys, où le dernier acide aminé est muté en Lys; ou bien mutée de manière à contrôler la cinétique de clivage de ce propeptide en utilisant le propeptide-Gly, issu du propeptide de Der p1 mais après mutation de l'asparagine du site de glycosylation en glutamine. De préférence, le propeptide peut donc être choisi parmi le propeptide de Der p1 où le dernier acide aminé est muté en lysine, et le propeptide de Der p1 où l'asparagine du site de glycosylation est mutée en glutamine. Le propeptide peut également être choisi parmi les propeptides de peptidases C1A d'origine végétale.

Une fois que le gène d'intérêt a été isolé et modifié pour contenir tout ou partie des modifications décrites ci-dessus et obtenir la séquence nucléotidique hétérologue, cette dernière est placée dans un vecteur d'expression par des méthodes classiques. La sélection d'un vecteur d'expression approprié dépendra de la méthode d'introduction du vecteur d'expression dans des cellules hôtes. Un vecteur d'expression typique contient des éléments d'ADN procaryote codant pour une origine de réplication bactérienne et un gène de résistance à un antibiotique à fournir pour la croissance et la sélection du vecteur d'expression dans l'hôte bactérien, un site de clonage pour l'insertion d'une séquence d'ADN exogène codant pour la protéase; des éléments d'ADN eucaryotes comme une séquence d'initiation de la transcription du gène exogène, comme un promoteur, et des éléments d'ADN qui contrôlent le traitement des transcrits, comme des séquences de terminaison / polyadénylation et une cassette d'expression permettant l'expression d'un inhibiteur du silencing. Il contient également des séquences telles que des t-DNA qui sont nécessaires pour l'intégration d'un morceau d'ADN dans la plante ou dans la cellule végétale.
De préférence, le vecteur d'expression comprend :
- des éléments d'ADN procaryote codant pour une origine de réplication bactérienne et un gène de résistance à un antibiotique ;
- au moins une séquence nucléotidique hétérologue codant pour une préproprotéine d'une peptidase C1A liée de manière fonctionnelle à un promoteur fort, de préférence un promoteur 35S ;
- une cassette d'expression permettant l'expression d'un inhibiteur du silencing, de préférence p19 ; et
- des éléments d'ADN qui contrôlent le traitement des transcrits, comme des séquences de terminaison / polyadénylation, de préférence la séquence Tnos (séquence de terminaison de la nopaline synthase).

De préférence, le vecteur d'expression est pAG01.

Les promoteurs utilisés pour contrôler l'expression de la protéase sont des promoteurs forts, et peuvent être des promoteurs de gènes de plantes, tels que par exemple, le promoteur de l'ubiquitine, le promoteur de la petite sous-unité de la ribulose-1, 5-bis-phosphate carboxylase, des promoteurs d'Agrobacterium tumefaciens, les promoteurs de la nopaline-synthase et de l'octopine synthase, ou encore des promoteurs viraux comme les 19S et 35S du virus de la mosaïque du chou-fleur (CaMV). De préférence, le promoteur fort est le 35S.

L'expression élevée et la qualité des peptidases recombinantes produites dans l'invention permettent de concevoir une production commerciale des protéases C1A. En effet, la peptidase C1A est typiquement exprimée en une quantité égale à au moins 0,1 % des protéines solubles totales de la plante, mais souvent en une quantité beaucoup plus élevée, pouvant atteindre 5 à 10%.

La plante comprenant les cellules végétales selon l'invention peut être une plante entière, mais peut également être une partie de plante, telle que des feuilles.

La plante ou les cellules végétales selon l'invention peuvent être utilisées en tant que telles, comme médicament. A titre d'exemple, lorsque la protéase C1A est utilisée comme une enzyme digestive, il peut être particulièrement avantageux de nourrir directement l'animal par la plante exprimant cette protéase. Dans d'autres applications, la protéase recombinante est purifiée après extraction à partir de la plante ou des cellules végétales qui l'expriment. Afin de faciliter sa purification, la protéase pourra être exprimée en fusion avec des tags (His6, GST, MBP, FLAG etc..) qui seront localisés de préférence en position N- ou C-terminale de la protéine mature.

La peptidase C1A susceptible d'être obtenue par le procédé selon l'invention, avec une cellule végétale selon l'invention, ou avec une plante selon l'invention, peut être utilisée comme médicament. Elle peut également être utilisée dans le diagnostic d'allergies, notamment d'allergies aux acariens ou d'allergies à certaines plantes. L'invention a donc également pour objet un kit de diagnostic d'allergies, comprenant la peptidase selon l'invention, et des moyens de mesure des anticorps dirigés contre cette peptidase.

Les méthodes générales de culture de plantes, ainsi que les procédés pour introduire des vecteurs d'expression dans un tissu végétal, sont à la disposition de l'homme de l'art. Ils sont variés et dépendent de la plante sélectionnée. De préférence, les plantes seront cultivées selon les techniques propres à la plateforme Allergopur. Ce procédé de production de protéines recombinantes est décrit dans la demande FR1255510, et comprend une première étape de culture de la plante, en aéroponie ou en hydroponie, de préférence culture sur flotteur libre, et sous éclairage LEDs. Après cette première étape, notamment cinq semaines de culture en hydroponie sur des flotteurs libres, l'agroinfiltration des plantes est réalisée sous vide, par des agrobactéries comprenant un fragment d'ADN codant pour la protéase C1A recombinante. Cette étape d'agroinfiltration peut être mise en oeuvre par tout moyen permettant de faire le vide. De préférence, dans le procédé utilisé selon l'invention, elle est réalisée sous vide par effet Venturi. Parmi les agrobactéries utilisables selon l'invention, on peut citer de préférence, les souches LBA4404, GV3101, EHA 101/105 ou C58. Une fois l'étape d'agroinfiltration réalisée, les plantes sont typiquement égouttées tête en bas pendant 15 minutes, puis remises en culture, typiquement 4 à 6 jours, idéalement en assurant une brumisation fréquente de ces dernières pendant les 6 premières heures de culture qui suivent l'agroinfiltation. Enfin, la protéine est extraite et purifiée.

Ainsi, de préférence, l'invention a également pour objet un procédé de production d'une peptidase C1A dans une cellule végétale ou une plante, comprenant les étapes suivantes :
a) transformation d'agrobactéries avec un vecteur d'expression comprenant une séquence nucléotidique hétérologue codant pour une préproprotéine d'une peptidase C1A liée de manière fonctionnelle à un promoteur fort ; et
b) transfection de la cellule végétale ou de la plante avec les agrobactéries obtenues à l'étape a).

De préférence, les agrobactéries utilisables dans l'étape a) sont choisies parmi les souches LBA4404, GV3101, EHA 101/105 et C58. De préférence, le vecteur d'expression utilisé dans l'étape a) comprend :
- des éléments d'ADN procaryote codant pour une origine de réplication bactérienne et un gène de résistance à un antibiotique ;
- au moins une séquence nucléotidique hétérologue codant pour une préproprotéine d'une peptidase C1A liée de manière fonctionnelle à un promoteur fort, de préférence un promoteur 35S ;
- une cassette d'expression permettant l'expression d'un inhibiteur du silencing, de préférence p19 ; et
- des éléments d'ADN qui contrôlent le traitement des transcrits, comme des séquences de terminaison / polyadénylation, de préférence la séquence Tnos.

La transformation de l'étape a) se fait typiquement par des méthodes connues de l'art antérieur, par exemple par chocs thermiques avec passages successifs à 4°C, -80°C et 37°C.

La transfection de l'étape b) comprend de préférence les étapes suivantes :
b1) culture de la cellule végétale ou de la plante, en aéroponie ou en hydroponie, et sous éclairage LEDs, de préférence pendant cinq semaines en hydroponie sur des flotteurs libres,
b2) agroinfiltration de la cellule végétale ou plante obtenue en b1) sous vide, par les agrobactéries obtenues à l'étape a). Cette étape d'agroinfiltration est, de préférence, réalisée sous vide par effet Venturi.
b3) remise en culture de la cellule végétale ou de la plante obtenue en b2), typiquement pendant 4 à 6 jours.

Enfin, de préférence, la peptidase C1A ainsi produite est extraite et purifiée.

Les exemples qui suivent, illustrent, mais ne visent pas à limiter la portée de l'invention. Il sera évident pour l'homme de l'art que des variantes et modifications sont possibles et entrent dans le cadre et l'esprit de l'invention.

Les légendes des figures sont les suivantes :

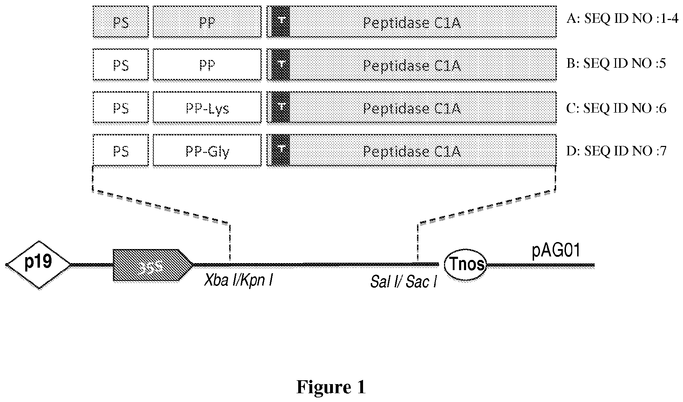

**Figure 1** **:** Représentation schématique des différentes cassettes permettant de produire une peptidase C1A mature et active en utilisant soit le peptide signal et le propeptide naturel de la protéine (A), soit le peptide signal de la chitinase de tabac et le propeptide de Der p1 (appelé aussi DP1) (P08176) (SEQ ID NO :8) afin d'augmenter les rendements (B), soit le peptide signal de la chitinase de tabac et le propeptide Der p1 modifié par insertion d'une lysine en position C-terminale du propeptide (SEQ ID NO :9) pour contrôler le clivage du propeptide (C) ou encore le peptide signal de la chitinase de tabac et le propeptide de Der p1 muté sur le site de N-glycosylation afin de contrôler la cinétique de clivage du propeptide (D) .
   A (SEQ ID NO :1-4): ADNc codant pour la forme naturelle de la protéine. Cet ADNc pourra être fusionné à des signaux d'adressage décrits dans la demande WO2008/056265,
   B (SEQ ID NO :5): ADNc codant pour la forme mature d'une peptidase C1A fusionné à la séquence signal de la chitinase de tabac (Neuhaus, J.-M. (1996) et au propeptide Der p1 (p08176 - aa 19 à 98 ou SEQ ID NO :8). Cet ADNc pourra être fusionné à des signaux d'adressage décrits dans la demande WO2008/056265,
   C (SEQ ID NO :6): ADNc codant pour la forme mature d'une peptidase C1A fusionné à la séquence signal de la chitinase de tabac (Neuhaus, J.-M. (1996)) et au propeptide Der p1 (p08176 - AA 19 à 98 ou SEQ ID NO :8) où l'acide aminé 98 de la séquence p08176 (= acide aminé 80 de SEQ ID NO :8) est substitué par une Lys (SEQ ID NO :9). Cet ADNc pourra être fusionné à des signaux d'adressage décrits dans la demande WO2008/056265,
   D (SEQ ID NO :7) : ADNc codant pour la forme mature d'une peptidase C1A fusionné à la séquence signal de la chitinase de tabac (Neuhaus, J.-M. (1996) et au propeptide Der p1 (p08176 - AA 19 à 98 ou SEQ ID NO :8) où l'asparagine 34 du site de glycosylation de la séquence p08176 (= acide aminé 16 de SEQ ID NO :8 ou 9) est mutée en glutamine. Cet ADNc pourra être fusionné à des signaux d'adressage décrits dans la demande WO2008/056265.

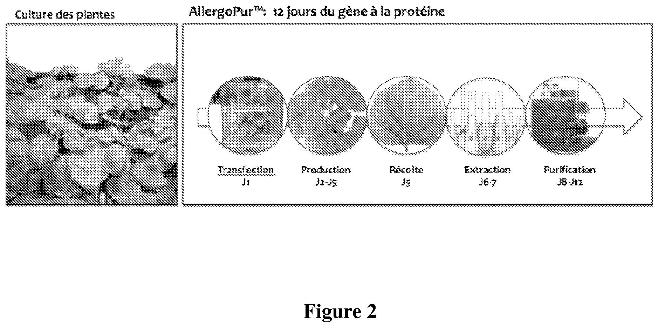

**Figure 2** **:** La plateforme AllergoPur utilisée pour l'expression et la production des différentes formes de peptidases C1A.

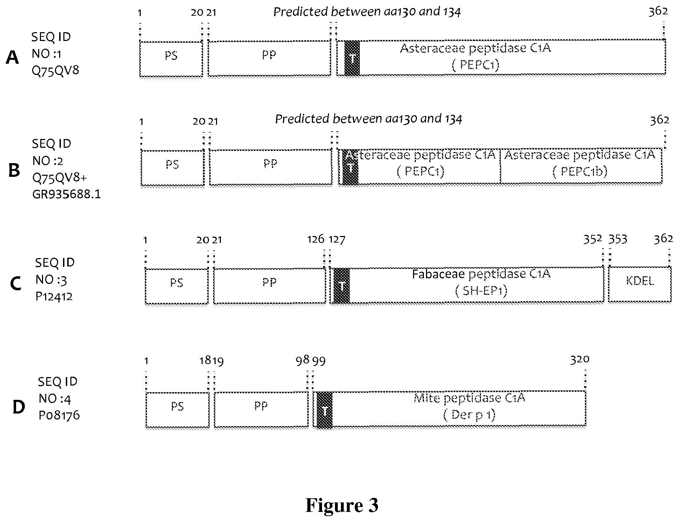

**Figure 3** **:** Représentation schématique de l'ADNc codant la forme précurseur de la peptidase C1A d'Aster tripolium (A), Vigna mungo (C) ou d'acariens (D) ou codant une forme précurseur chimérique de la peptidase C1A d'Asteracées (B). Les gènes sont exprimés sous dépendance du promoteur 35S, et en amont de la séquence de terminaison de transcription de la nopaline synthase. Le plasmide comprend les séquences nécessaires à l'incorporation dans la plante en utilisant Agrobacterium, comprenant les limites gauche et droite de l'ADN-T, la région d'origine de réplication, et le site de co-intégration. Un TAG sera ou non intégré en position N- ou C-terminale de la protéine mature.

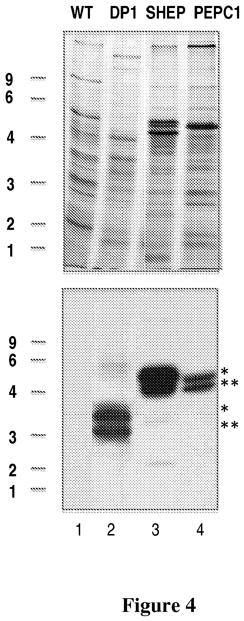

**Figure 4****:** Les protéines extraites de plantes non transfectées (WT piste 1) ou transfectées sous vide pour l'expression des protéines DP1 (piste 2), SHEP (piste 3) ou PEPC1 (piste 4) ont été analysées par SDS-PAGE (panneau A) et immmunodétection avec un anticorps dirigé contre une étiquette Flag (panneau B). L'analyse par immunodétection met en évidence la production spécifique des 3 protéases de la famille C1A. Ces protéases sont exprimées sous la forme précurseur (*) et sous la forme mature (**).

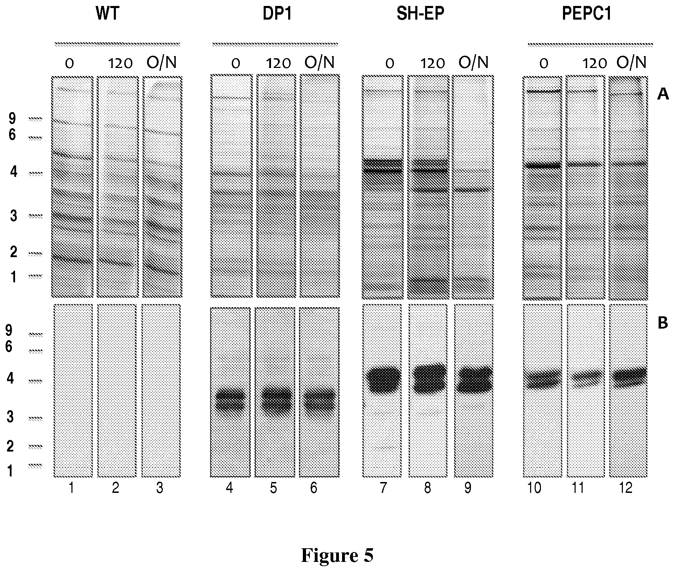

**Figure 5****:** Les protéines extraites de plantes non transfectées (WT piste 1), transfectées sous vide avec la protéine DP1 (piste 2), SHEP (piste 3) ou PEPC1 (piste 4) ont été analysées par SDS-PAGE (panneau A) et immmunodétection avec un anticorps dirigé contre une étiquette Flag (panneau B). Les protéines ont été extraites en condition native puis incubées à 4°C à 0 min, 120 min ou toute la nuit (O/N). L'analyse SDS-PAGE met en évidence la protéolyse de plusieurs protéines endogènes des extraits de plantes exprimant une protéase C1A alors que ces protéines sont stables dans les extraits contrôles (WT) (Panneau A). La protéase C1A contenue dans ces extraits est stable dans les mêmes conditions (panneau B).

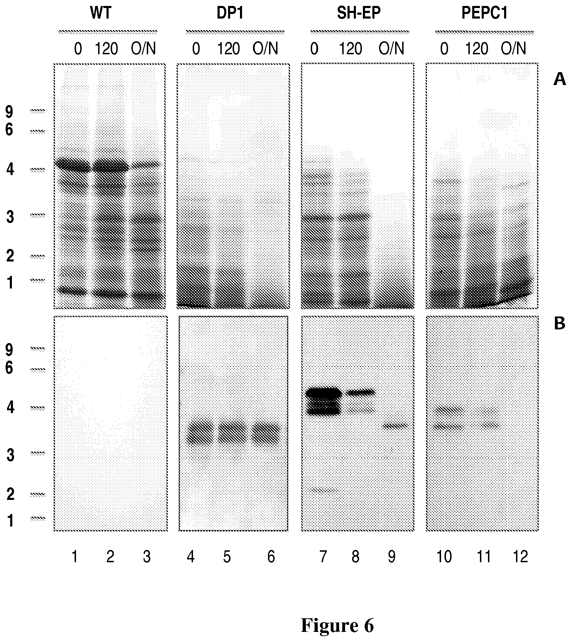

**Figure 6** **:** Les protéines extraites de plantes non transfectées (WT piste 1), transfectées sous vide avec la protéine DP1 (piste 2), SHEP (piste 3) ou PEPC1 (piste 4) ont été analysées par SDS-PAGE (panneau A) et immmunodétection avec un anticorps dirigé contre une étiquette Flag (panneau B). Les protéines ont été extraites en condition dénaturante en présence d'un agent réducteur puis incubées à 4°C à 0 min, 120 min ou toute la nuit (O/N). Dans ces conditions, l'analyse SDS-PAGE met en évidence une forte dégradation des protéines endogènes des extraits de plantes exprimant une protéase C1A alors que ces protéines sont stables dans les extraits contrôles (WT). Cette dégradation est quasi totale pour les extraits incubés sur la nuit (Panneau A). Dans les mêmes conditions certaines protéases C1A sont également dégradées au cours du temps comme l'illustre l'analyse par immunodétection (panneau B).

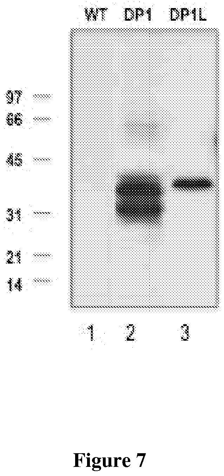

**Figure 7** : Les protéines extraites de plantes non transfectées (WT piste 1) ou transfectées sous vide avec la protéine DP1 (piste 2), ou par la protéine Derp1L (DP1L) dont le propeptide a été muté par substitution de Gln 98 de la séquence p08176 par une lysine (propeptide de séquence SEQ ID NO :9) (piste 3) ont été analysées par SDS-PAGE et immmunodétection avec un anticorps dirigé contre une étiquette Flag. L'analyse par immunodétection met en évidence la production la forme précurseur et la forme mature pour les plantes exprimant la protéine DP1, alors que seule la forme précurseur inactive est produite chez les plantes exprimant DP1L.

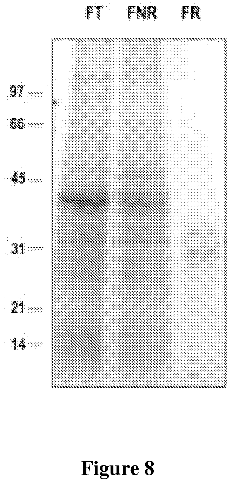

**Figure 8****:** Les protéines extraites de plantes transfectées sous vide avec la protéine DP1 ont été purifiées sur colonne GE Healthcare HisTrap Excell puis les fractions totale (FT), non retenue sur la colonne (FNR) ou retenue sur la colonne (FR) ont été analysées par électrophorèse SDS_PAGE. Comme l'illustre la piste FR, la protéase DP1 est purifiée à 85%, cette étape est suivie d'une étape de gel filtration pour parfaire la purification.

### EXEMPLE :

### Design moléculaire et synthèse de gène

Les ADNc sont synthétisés en optimisant l'usage des codons pour leur reconnaissance par le système végétal. Dans le cadre de cette invention, l'optimisation préférée est l'optimisation pour une expression chez *Nicotiana benthamiana,* comme indiqué en Figure 1.

### Préparation des plasmides

Des sites de restriction Xba I /kpn I et Sal I / Sac I sont respectivement intégrés aux extrémités 5' et 3' de l'ADNc lors de la synthèse. Ces sites sont ensuite utilisés afin de cloner les ADNc dans le vecteur binaire d'expression pAG01 (Figure 1). Les ADNc sont clonés en amont d'un promoteur 35S (35S) et en aval d'une séquence de terminaison de la nopaline synthase (Tnos) ; le vecteur pAG01 contient en outre une cassette d'expression permettant d'exprimer l'inhibiteur de silencing p19 simultanément de la protéine recombinante afin d'augmenter les rendements de production. Les vecteurs sont ensuite utilisés pour transformer la souche LBA4404 *d'Agrobacterium tumefaciens.*

### Expression transitoire des peptidases C1A dans des feuilles de Nicotiana benthamiana - Utilisation de la plateforme AllergoPur

Pour la production par expression transitoire *Agobacterium tumefaciens LBA4404* est utilisé pour le transfert d'un ADNc codant la protéase sans que le gène d'intérêt soit intégré dans le génome de la cellule végétale : on parle ici de transfection et non de transgenèse. Les plantes sont cultivées en condition hydroponique en présence d'un milieu nutritif (GHE, floragrow, floramicro, florabloom, 10mL/ 15mL/ 5mL pour 10 L d'EAU osmose) et sous éclairage LED. L'agrobactérie est transférée dans le tissu foliaire par agro-infiltration selon deux procédés. Pour la production de petits lots de protéases destinés au criblage de prototypes, les agrobactéries sont injectées manuellement grâce à une seringue appliquée contre l'épiderme de la face inférieure de la feuille. Des disques foliaires prélevés dans les feuilles 4 à 6 jours après l'agro-infiltration sont utilisés pour l'analyse des différents prototypes de protéases. Cette étape de criblage permet de définir le vecteur d'expression qui sera utilisé pour l'obtention d'une protéase de qualité optimale. Le même procédé est utilisé pour la production commerciale à grande échelle mais dans ce cas, l'agro-infiltration s'effectue sous vide, dans des enceintes contenant plusieurs litres d'une culture d'agrobactéries et où plusieurs dizaines de plantes sont infiltrées simultanément. Ces plantes sont ensuite remises en culture pendant 4 à 6 jours avant la purification de la protéase C1A à partir des extraits foliaires (Figure 2).

### Expression de 4 peptidases de la famille C1

L'expression des protéines ainsi que les rendements sont respectivement analysés par Western blotting et ELISA. Les résultats sont illustrés pour 3 peptidases C1A de plantes (SH-EP-P12412 et PEPC1-Q75QV8) ou d'acarien (DP1- p08176) (Figures 3 et 4).

Les protéases exprimées sont actives. En effet, les protéines végétales extraites à partir des feuilles exprimant les protéases C1A sont rapidement dégradées après l'extraction, alors qu'elles sont stables dans les extraits de plantes non transfectées (Figure 5). De plus, cette dégradation des protéines endogènes est beaucoup rapide et incontrôlée lorsque l'extraction est réalisée en présence d'agents réducteurs (Figure 6). Cette activité accrue des peptidases C1A en présence des agents réducteurs est une caractéristique de ces protéases (Beers et al, 2000, Plant Mol Biol, 44: 399-415).

### Production d'une protéase C1A sous la forme d'un précurseur inactif

La fusion de l'ADNc codant la protéine mature de DP1 avec la séquence signal de la chitinase et le propeptide de séquence SEQ ID NO :9 (modifié par la substitution glutamine 80 / lysine en position C-terminale du propeptide) (séquence totale DP1L, SEQ ID NO :6) permet comme l'illustre la figure 7 de bloquer le clivage du propeptide et d'accumuler une protéine inactive dans l'extrait végétal. Ce propeptide peut ensuite être clivé *in vivo* lors d'une co-expression de la protéine Lys-C ou in vitro en incubation avec la même protéine.

### Purification et caractérisation

Comme l'illustre la figure 8, les protéases sont extraites à partir de la biomasse fraîche ou congelée puis purifiées sur colonne HisTrap Excell.

Comme l'illustre la piste FR, la protéase DP1 est purifiée à 85%, cette étape est suivie d'une étape de gel filtration pour parfaire la purification.

## Revendications

1. Cellule végétale comprenant une molécule d'ADN comprenant au moins une séquence nucléotidique hétérologue codant pour une préproprotéine d'une peptidase C1A liée de manière fonctionnelle à un promoteur fort, dans laquelle ladite molécule d'ADN n'est pas intégrée dans le génome de la cellule végétale.

2. Cellule végétale selon la revendication 1, dans laquelle la peptidase est choisie parmi les peptidases C1A d'acariens et les peptidases C1A végétales.

3. Cellule végétale selon la revendication 1 ou 2, dans laquelle la peptidase C1A est choisie parmi Der p 1, Der f1, Eur m 1, PEPC1, SH-EP, AMB, AMB A11, la séquence SEQ ID NO :10, la séquence SEQ ID NO :11, la séquence SEQ ID NO :12 et les protéines présentant au moins 70% d'identité avec l'une de ces dernières.

4. Cellule végétale selon l'une des revendications 1 à 3, dans laquelle la peptidase C1A est exprimée en une quantité égale à au moins 0,1 % des protéines solubles totales de la plante.

5. Cellule végétale selon l'une des revendications 1 à 4, dans laquelle la séquence nucléotidique hétérologue comprend une séquence codant le peptide signal, une séquence codant le propeptide et une séquence codant la protéine mature C1A.

6. Cellule végétale selon la revendication 5, dans laquelle la séquence codant le peptide signal est choisie parmi le peptide signal naturel de la peptidase et le peptide signal de la chitinase de tabac ; et le propeptide est choisi parmi les propeptides naturels de peptidases C1A, le propeptide naturel de SH-EP (séquence SEQ ID NO :13), la séquence SEQ ID NO :8, la séquence SEQ ID NO :9 et la séquence SEQ ID NO :8 ou 9 mutée sur l'asparagine 16.

7. Cellule végétale selon l'une des revendications 1 à 6, dans laquelle la séquence nucléotidique hétérologue comprend en outre une séquence peptidique d'adressage qui cible la peptidase C1A sous forme soluble ou membranaire vers le réticulum endoplasmique ou les différents compartiments constitutifs du système endomembranaire de sécrétion de la cellule végétale.

8. Cellule végétale selon l'une des revendications 1 à 7, qui comprend un vecteur d'expression comprenant :
- des éléments d'ADN procaryote codant pour une origine de réplication bactérienne et un gène de résistance à un antibiotique ;
- la séquence nucléotidique hétérologue telle que définie dans l'une des revendications précédentes ;
- une cassette d'expression permettant l'expression d'un inhibiteur du silencing, de préférence p19 ; et
- des éléments d'ADN qui contrôlent le traitement des transcrits, comme des séquences de terminaison / polyadénylation, de préférence la séquence Tnos.

9. Plante comprenant au moins une cellule végétale selon l'une des revendications 1 à 8.

10. Procédé de production d'une peptidase C1A, comprenant l'expression de ladite peptidase C1A dans une cellule végétale selon l'une des revendications 1 à 8, ou dans une plante selon la revendication 9.

11. Procédé de production d'une peptidase C1A selon la revendication 10, comprenant les étapes suivantes :
a) transformation d'agrobactéries avec un vecteur d'expression comprenant une séquence nucléotidique hétérologue codant pour une préproprotéine d'une peptidase C1A liée de manière fonctionnelle à un promoteur fort ; et
b) transfection de la cellule végétale ou de la plante avec les agrobactéries obtenues à l'étape a).

12. Procédé de production d'une peptidase C1A selon la revendication 10 ou 11, dans lequel les agrobactéries utilisées dans l'étape a) sont choisies parmi les souches LBA4404, GV3101, EHA 101/105 et C58, et dans lequel la transfection de l'étape b) comprend de préférence les étapes suivantes :
b1) culture de la cellule végétale ou de la plante, en aéroponie ou en hydroponie, et sous éclairage LEDs, de préférence pendant cinq semaines en hydroponie sur des flotteurs libres,
b2) agroinfiltration de la cellule végétale ou plante obtenue en b1) sous vide, par les agrobactéries obtenues à l'étape a) ; et
b3) remise en culture de la cellule végétale ou de la plante obtenue en b2), typiquement pendant 4 à 6 jours.

13. Utilisation d'une peptidase obtenue par le procédé selon l'une des revendications 10 à 12 dans une méthode de diagnostic d'allergies, notamment d'allergies aux acariens ou d'allergies à certaines plantes.

## Patentansprüche

1. Pflanzenzelle, umfassend ein DNA-Molekül mit mindestens einer heterologen Nukleotidsequenz, die ein Präproprotein einer ClA-Peptidase kodiert, das funktionell an einen starken Promotor gebunden ist, wobei das DNA-Molekül nicht in das Genom der Pflanzenzelle integriert ist.

2. Pflanzenzelle gemäß Anspruch 1, wobei die Peptidase aus Milben-ClA-Peptidasen und Pflanzen-ClA-Peptidasen ausgewählt ist.

3. Pflanzenzelle gemäß Anspruch 1 oder 2, wobei die C1A-Peptidase ausgewählt ist aus Der p1, Der f1, Eur m1, PEPCl, SH-EP, AMB, AMB A11, der Sequenz SEQ ID NO: 10, der Sequenz SEQ ID NO: 11, der Sequenz SEQ ID NO: 12 und Proteinen, die mindestens zu 70% mit einem der vorgenannten identisch sind.

4. Pflanzenzelle gemäß einem der Ansprüche 1 bis 3, wobei die C1A-Peptidase in einer Menge exprimiert ist, die mindestens 0,1% der gesamten löslichen Proteine der Pflanze entspricht.

5. Pflanzenzelle gemäß einem der Ansprüche 1 bis 4, wobei die heterologe Nukleotidsequenz eine Sequenz, die das Signalpeptid kodiert, eine Sequenz, die das Propeptid kodiert und eine Sequenz, die das reife ClA-Protein kodiert, umfasst.

6. Pflanzenzelle gemäß Anspruch 5, wobei die Sequenz, die das Signalpeptid kodiert aus dem natürlichen Signalpeptid der Peptidase und dem Signalpeptid der Tabakchitinase ausgewählt ist; und das Propeptid aus den natürlichen Propeptiden der ClA-Peptidasen, dem natürlichen Propeptid von SH-EP (Sequenz SEQ ID NO: 13), der Sequenz SEQ ID NO: 8, der Sequenz SEQ ID NO: 9 und der auf Asparagin 16 mutierten Sequenz SEQ ID NO: 8 oder 9 ausgewählt ist.

7. Pflanzenzelle gemäß einem der Ansprüche 1 bis 6, wobei die heterologe Nukleotidsequenz ferner eine Target-Peptidsequenz umfasst, die die ClA-Peptidase in löslicher Form oder in Membranform auf das endoplasmatischen Retikulum oder verschiedene Kompartimente, die das Endomembran-Sekretionssystem der Pflanzenzelle bilden, abzielt.

8. Pflanzenzelle gemäß einem der Ansprüche 1 bis 7 mit einem Expressionsvektor umfassend:
prokaryotische DNA-Elemente, die eine bakterielle Replikationsquelle und ein gegen Antibiotika resistentes Gen kodieren;
die wie in einem der vorherigen Ansprüche definierte heterologe Nukleotidsequenz;
eine Expressionskassette, die die Expression eines Stilllegungsinhibitors ermöglicht, vorzugsweise p19; und
DNA-Elemente, die die Verarbeitung von Transkriptionen steuern, wie die Terminationssequenzen / die Polyadenylierungssequenzen, vorzugsweise die Tnos-Sequenz.

9. Pflanze, enthaltend mindestens eine Pflanzenzelle gemäß einem der Ansprüche 1 bis 8.

10. Verfahren zur Herstellung einer ClA-Peptidase, umfassend die Expression der ClA-Peptidase in einer Pflanzenzelle gemäß einem der Ansprüche 1 bis 8 oder in einer Pflanze gemäß Anspruch 9.

11. Verfahren zur Herstellung einer ClA-Peptidase gemäß Anspruch 10, umfassend die folgenden Schritte:
a) Transformieren von Agrobakterien mit einem Expressionsvektor, umfassend eine heterologe Nukleotidsequenz, die das Präproprotein einer C1A-Peptidase kodiert, der funktionell an einem starken Promotor gebunden ist; und
b) Transfizieren der Pflanzenzelle oder Pflanze mit den in Schritt a) erhaltenen Agrobakterien.

12. Verfahren zur Herstellung einer ClA-Peptidase gemäß Anspruch 10 oder 11, wobei die in Schritt a) verwendeten Agrobakterien aus den Stämmen LBA4404, GV3101, EHA 101/105 und C58 ausgewählt werden und wobei das Transfizieren in Schritt b) vorzugsweise die folgenden Schritte umfasst:
b1) aeroponisches oder hydroponisches Kultivieren der Pflanzenzelle oder Pflanze unter LED-Beleuchtung, vorzugsweise fünf Wochen in Hydrokultur auf freien Schwimmkörpern,
b2) Agrofiltrieren der in b1) erhaltenen Pflanzenzelle oder Pflanze durch die in Schritt a) erhaltenen Agrobakterien unter Vakuum; und
b3) Rekultivieren der in b2) erhaltenen Pflanzenzelle oder Pflanze, typischerweise für 4 bis 6 Tage.

13. Verwendung einer durch das Verfahren gemäß einem der Ansprüche 10 bis 12 erhaltenen Peptidase in einem Verfahren zur Diagnose von Allergien, insbesondere Allergien gegen Milben oder Allergien gegen bestimmte Pflanzen.

## Claims

1. Plant cell comprising a DNA molecule comprising at least one heterologous nucleotide sequence coding for a preproprotein of a C1A peptidase functionally linked to a strong promoter, wherein said DNA molecule is not integrated in the genome of the plant cell.

2. Plant cell according to claim 1, wherein the peptidase is chosen from the group consisting of mite C1A peptidases and plant C1A peptidases.

3. Plant cell according to either claim 1 or claim 2, wherein the C1A peptidase is chosen from the group consisting of Der p 1, Der f1, Eur m 1, PEPC1, SH-EP, AMB, AMB A11, the sequence SEQ ID NO: 10, the sequence SEQ ID NO: 11, the sequence SEQ ID NO: 12 and the proteins having at least 70% identity to one thereof.

4. Plant cell according to one of claims 1 to 3, wherein the C1A peptidase is expressed as a quantity equal to at least 0.1% of the total soluble proteins of the plant.

5. Plant cell according to one of claims 1 to 4, wherein the heterologous nucleotide sequence comprises a sequence encoding the signal peptide, a sequence encoding the propeptide, and a sequence encoding the mature C1A protein.

6. Plant cell according to claim 5, wherein the sequence encoding the signal peptide is chosen from the group consisting of the natural signal peptide of the peptidase and the signal peptide of the chitinase of tobacco; and the propeptide is chosen from the group consisting of the natural propeptides of C1A peptidases, the natural propeptide of SH-EP (sequence SEQ ID NO: 13), the sequence SEQ ID NO: 8, the sequence SEQ ID NO: 9 and the sequence SEQ ID NO: 8 or 9 mutated on asparagine 16.

7. Plant cell according to one of claims 1 to 6, wherein the heterologous nucleotide sequence further comprises a peptide addressing sequence which targets the C1A peptidase in soluble or membrane form to the endoplasmic reticulum or the different compartments constituting the secretory endomembrane system of the plant cell.

8. Plant cell according to one of claims 1 to 7, comprising an expression vector comprising:
- prokaryotic DNA elements coding for a bacterial replication origin and an antibiotic resistance gene;
- the heterologous nucleotide sequence as defined in one of the previous claims;
- an expression cassette allowing for the expression of a silencing suppressor, preferably p19; and
- DNA elements that control transcript processing, such as termination/polyadenylation sequences, preferably the Tnos sequence.

9. Plant comprising at least one plant cell according to one of claims 1 to 8.

10. Method for producing a C1A peptidase, comprising the expression of said C1A peptidase in a plant cell according to one of claims 1 to 8, or in a plant according to claim 9.

11. Method for producing a C1A peptidase according to claim 10, comprising the following steps of:
a) transforming agrobacteria with an expression vector comprising a heterologous nucleotide sequence coding for a preproprotein of a C1A peptidase functionally linked to a strong promoter; and
b) transfecting the plant cell or plant with the agrobacteria obtained in step a).

12. Method for producing a C1A peptidase according to claim 10 or 11, wherein the agrobacteria used in step a) are chosen from the group consisting of the strains LBA4404, GV3101, EHA 101/105 and C58, and wherein the transfection of step b) preferably comprises the following steps of:
b1) growing the plant cell or plant, in aeroponic or hydroponic conditions, and under LED lighting, preferably for five weeks under hydroponic conditions on free floats,
b2) agroinfiltrating the plant cell or plant obtained in b1) under a vacuum, by the agrobacteria obtained in step a); and
b3) re-growing the plant cell or plant obtained in b2), generally for 4 to 6 days.

13. Use of a peptidase obtained by the method according to one of claims 10 to 12 in a method for diagnosing allergies, in particular mite allergies or allergies to certain plants.


### SEQUENCE LISTING

<110> ANGANY GENETICS
<120> Production commerciale de peptidases C1A par expression transitoire chez les plantes
<130> BFF130140
<160> 16
<170> PatentIn version 3.5
<210> 1
   <211> 363
   <212> PRT
   <213> Artificial Sequence
<220>
   <223> Q75QV8
<400> 1
   Met Asn Leu Leu Ser Ile Ser Ser Leu Leu Leu Leu Leu Leu Ile Ser
   1               5                   10                  15
   Ala Val Thr Ala Asp Ser Ser Asp Pro Leu Ile Arg Gln Val Val Gln
               20                  25                  30
   Asn Asp Glu Thr Glu Ile Glu Ser Asp Pro Leu Leu Asp Pro Glu His
           35                  40                  45
   His Phe Lys Leu Phe Lys Asn Lys Phe Gly Arg Thr Tyr Asp Thr Glu
       50                  55                  60
   Glu Glu His Glu Tyr Arg Leu Thr Val Phe Lys Ser Asn Leu Arg Arg
   65                  70                  75                  80
   Ala Lys Arg His Gln Val Leu Asp Pro Thr Ala Lys His Gly Val Thr
                   85                  90                  95
   Lys Phe Ser Asp Leu Thr Pro Ser Glu Phe Arg Lys Lys Tyr Leu Gly
               100                 105                 110
   Leu Lys Ser Lys Leu Lys Leu Pro Ala Asp Ala Asn Lys Ala Pro Ile
           115                 120                 125
   Leu Pro Thr Ser Asn Leu Pro Gln Asp Phe Asp Trp Arg Asp Lys Gly
       130                 135                 140
   Ala Val Thr Pro Val Lys Asn Gln Gly Ser Cys Gly Ser Cys Trp Ser
   145                 150                 155                 160
   Phe Ser Thr Thr Gly Ala Leu Glu Gly Ser His Phe Leu Gln Thr Gly
                   165                 170                 175
   Glu Leu Val Ser Leu Ser Glu Gln Gln Leu Val Asp Cys Asp His Glu
               180                 185                 190
   Cys Asp Pro Ala Glu Tyr Asn Ser Cys Asp Ser Gly Cys Asn Gly Gly
           195                 200                 205
   Leu Met Asn Asn Ala Phe Glu Tyr Ile Leu Lys Ala Gly Gly Leu Gln
       210                 215                 220
   Lys Glu Ala Asp Tyr Pro Tyr Thr Gly Arg Asp Gly Thr Cys Lys Phe
   225                 230                 235                 240
   Asp Lys Ser Lys Ile Ala Ala Ser Val Ala Asn Phe Ser Val Val Ser
                   245                 250                 255
   Thr Asp Glu Asp Gln Ile Ala Ala Asn Leu Val Thr Asn Gly Pro Leu
               260                 265                 270
   Ala Ile Gly Ile Asn Ala Ala Trp Met Gln Thr Tyr Ile Gly Gln Val
           275                 280                 285
   Ser Cys Pro Tyr Ile Cys Ser Lys Thr Lys Met Asp His Gly Val Leu
       290                 295                 300
   Leu Val Gly Tyr Gly Ser Ala Gly Tyr Ala Pro Leu Arg Phe Lys Glu
   305                 310                 315                 320
   Lys Pro Tyr Trp Ile Ile Lys Asn Ser Trp Gly Glu Asp Trp Gly Glu
                   325                 330                 335
   Asp Gly Tyr Tyr Lys Leu Cys Ser Gly Tyr Asn Ala Cys Gly Met Asp
               340                 345                 350
   Thr Met Val Ser Ala Val Val Ser Thr Asn Thr
           355                 360
<210> 2
   <211> 363
   <212> PRT
   <213> Artificial Sequence
<220>
   <223> Q75QV8+GR935688.1
<400> 2
   Met Asn Leu Leu Ser Ile Ser Ser Leu Leu Leu Leu Leu Leu Ile Ser
   1               5                   10                  15
   Ala Val Thr Ala Asp Ser Ser Asp Pro Leu Ile Arg Gln Val Val Gln
               20                  25                  30
   Asn Asp Glu Thr Glu Ile Glu Ser Asp Pro Leu Leu Asp Pro Glu His
           35                  40                  45
   His Phe Lys Leu Phe Lys Asn Lys Phe Gly Arg Thr Tyr Asp Thr Glu
       50                  55                  60
   Glu Glu His Glu Tyr Arg Leu Thr Val Phe Lys Ser Asn Leu Arg Arg
   65                  70                  75                  80
   Ala Lys Arg His Gln Val Leu Asp Pro Thr Ala Lys His Gly Val Thr
                   85                  90                  95
   Lys Phe Ser Asp Leu Thr Pro Ser Glu Phe Arg Lys Lys Tyr Leu Gly
               100                 105                 110
   Leu Lys Ser Lys Leu Lys Leu Pro Ala Asp Ala Asn Lys Ala Pro Ile
           115                 120                 125
   Leu Pro Thr Ser Asn Leu Pro Gln Asp Phe Asp Trp Arg Asp Lys Gly
       130                 135                 140
   Ala Val Thr Pro Val Lys Asn Gln Gly Ser Cys Gly Ser Cys Trp Ser
   145                 150                 155                 160
   Phe Ser Thr Thr Gly Ala Leu Glu Gly Ser His Phe Leu Gln Thr Gly
                   165                 170                 175
   Glu Leu Val Ser Leu Ser Glu Gln Gln Leu Val Asp Cys Asp His Glu
               180                 185                 190
   Cys Asp Pro Ala Glu Tyr Asn Ser Cys Asp Ser Gly Cys Asn Gly Gly
           195                 200                 205
   Leu Met Asn Asn Ala Phe Glu Tyr Ile Leu Lys Ala Gly Gly Val Gln
       210                 215                 220
   Lys Glu Ser Asp Tyr Pro Tyr Thr Gly Lys Asp Gly Thr Cys His Phe
   225                 230                 235                 240
   Asp Lys Thr Lys Ile Ala Ala Ser Val Ser Asn Phe Ser Val Ile Gly
                   245                 250                 255
   Thr Asp Glu Asp Gln Ile Ala Ala Asn Leu Val Lys Asn Gly Pro Leu
               260                 265                 270
   Ala Ile Gly Ile Asn Ala Ala Trp Met Gln Thr Tyr Ile Gly Lys Val
           275                 280                 285
   Ser Cys Pro Tyr Ile Cys Ser Lys Lys Arg Leu Asp His Gly Val Leu
       290                 295                 300
   Leu Val Gly Tyr Gly Ser Ala Gly Tyr Ala Pro Ser Arg Leu Lys Glu
   305                 310                 315                 320
   Lys Pro Tyr Trp Ile Ile Lys Asn Ser Trp Gly Pro Asp Trp Gly Glu
                   325                 330                 335
   Asp Gly Tyr Tyr Lys Ile Cys Ser Gly Tyr Asn Leu Cys Gly Met Asp
               340                 345                 350
   Thr Met Val Ser Ala Val Val Ser Thr Asn Thr
           355                 360
<210> 3
   <211> 362
   <212> PRT
   <213> Artificial Sequence
<220>
   <223> P12412
<400> 3
   Met Ala Met Lys Lys Leu Leu Trp Val Val Leu Ser Leu Ser Leu Val
   1               5                   10                  15
   Leu Gly Val Ala Asn Ser Phe Asp Phe His Glu Lys Asp Leu Glu Ser
               20                  25                  30
   Glu Glu Ser Leu Trp Asp Leu Tyr Glu Arg Trp Arg Ser His His Thr
           35                  40                  45
   Val Ser Arg Ser Leu Gly Glu Lys His Lys Arg Phe Asn Val Phe Lys
       50                  55                  60
   Ala Asn Val Met His Val His Asn Thr Asn Lys Met Asp Lys Pro Tyr
   65                  70                  75                  80
   Lys Leu Lys Leu Asn Lys Phe Ala Asp Met Thr Asn His Glu Phe Arg
                   85                  90                  95
   Ser Thr Tyr Ala Gly Ser Lys Val Asn His His Lys Met Phe Arg Gly
   100 105 110
   Ser Gln His Gly Ser Gly Thr Phe Met Tyr Glu Lys Val Gly Ser Val
   115 120 125
   Pro Ala Ser Val Asp Trp Arg Lys Lys Gly Ala Val Thr Asp Val Lys
   130 135 140
   Asp Gln Gly Gln Cys Gly Ser Cys Trp Ala Phe Ser Thr Ile Val Ala
   145 150 155 160
   Val Glu Gly Ile Asn Gln Ile Lys Thr Asn Lys Leu Val Ser Leu Ser
   165 170 175
   Glu Gln Glu Leu Val Asp Cys Asp Lys Glu Glu Asn Gln Gly Cys Asn
   180 185 190
   Gly Gly Leu Met Glu Ser Ala Phe Glu Phe Ile Lys Gln Lys Gly Gly
   195 200 205
   Ile Thr Thr Glu Ser Asn Tyr Pro Tyr Thr Ala Gln Glu Gly Thr Cys
   210 215 220
   Asp Glu Ser Lys Val Asn Asp Leu Ala Val Ser Ile Asp Gly His Glu
   225 230 235 240
   Asn Val Pro Val Asn Asp Glu Asn Ala Leu Leu Lys Ala Val Ala Asn
   245 250 255
   Gln Pro Val Ser Val Ala Ile Asp Ala Gly Gly Ser Asp Phe Gln Phe
   260 265 270
   Tyr Ser Glu Gly Val Phe Thr Gly Asp Cys Asn Thr Asp Leu Asn His
   275 280 285
   Gly Val Ala Ile Val Gly Tyr Gly Thr Thr Val Asp Gly Thr Asn Tyr
   290 295 300
   Trp Ile Val Arg Asn Ser Trp Gly Pro Glu Trp Gly Glu Gln Gly Tyr
   305 310 315 320
   Ile Arg Met Gln Arg Asn Ile Ser Lys Lys Glu Gly Leu Cys Gly Ile
   325 330 335
   Ala Met Met Ala Ser Tyr Pro Ile Lys Asn Ser Ser Asp Asn Pro Thr
   340 345 350
   Gly Ser Leu Ser Ser Pro Lys Asp Glu Leu
   355 360
<210> 4
   <211> 320
   <212> PRT
   <213> Artificial Sequence
<220>
   <223> P08176
<400> 4
   Met Lys Ile Val Leu Ala Ile Ala Ser Leu Leu Ala Leu Ser Ala Val
   1               5                   10                  15
   Tyr Ala Arg Pro Ser Ser Ile Lys Thr Phe Glu Glu Tyr Lys Lys Ala
               20                  25                  30
   Phe Asn Lys Ser Tyr Ala Thr Phe Glu Asp Glu Glu Ala Ala Arg Lys
           35                  40                  45
   Asn Phe Leu Glu Ser Val Lys Tyr Val Gln Ser Asn Gly Gly Ala Ile
       50                  55                  60
   Asn His Leu Ser Asp Leu Ser Leu Asp Glu Phe Lys Asn Arg Phe Leu
   65                  70                  75                  80
   Met Ser Ala Glu Ala Phe Glu His Leu Lys Thr Gln Phe Asp Leu Asn
                   85                  90                  95
   Ala Glu Thr Asn Ala Cys Ser Ile Asn Gly Asn Ala Pro Ala Glu Ile
               100                 105                 110
   Asp Leu Arg Gln Met Arg Thr Val Thr Pro Ile Arg Met Gln Gly Gly
           115                 120                 125
   Cys Gly Ser Cys Trp Ala Phe Ser Gly Val Ala Ala Thr Glu Ser Ala
       130                 135                 140
   Tyr Leu Ala Tyr Arg Asn Gln Ser Leu Asp Leu Ala Glu Gln Glu Leu
   145                 150                 155                 160
   Val Asp Cys Ala Ser Gln His Gly Cys His Gly Asp Thr Ile Pro Arg
                   165                 170                 175
   Gly Ile Glu Tyr Ile Gln His Asn Gly Val Val Gln Glu Ser Tyr Tyr
               180                 185                 190
   Arg Tyr Val Ala Arg Glu Gln Ser Cys Arg Arg Pro Asn Ala Gln Arg
                   195                 200                 205
   Phe Gly Ile Ser Asn Tyr Cys Gln Ile Tyr Pro Pro Asn Val Asn Lys
       210                 215                 220
   Ile Arg Glu Ala Leu Ala Gln Thr His Ser Ala Ile Ala Val Ile Ile
   225                 230                 235                 240
   Gly Ile Lys Asp Leu Asp Ala Phe Arg His Tyr Asp Gly Arg Thr Ile
                   245                 250                 255
   Ile Gln Arg Asp Asn Gly Tyr Gln Pro Asn Tyr His Ala Val Asn Ile
               260                 265                 270
   Val Gly Tyr Ser Asn Ala Gln Gly Val Asp Tyr Trp Ile Val Arg Asn
           275                 280                 285
   Ser Trp Asp Thr Asn Trp Gly Asp Asn Gly Tyr Gly Tyr Phe Ala Ala
       290                 295                 300
   Asn Ile Asp Leu Met Met Ile Glu Glu Tyr Pro Tyr Val Val Ile Leu
   305                 310                 315                 320
<210> 5
   <211> 334
   <212> PRT
   <213> Artificial Sequence
<220>
   <223> ADNc codant pour la forme mature d'une peptidase C1A fusionné à la séquence signal de la chitinase de tabac
<400> 5
   Met Lys Ile Val Leu Ala Ile Ala Ser Leu Leu Ala Leu Ser Ala Val
   1               5                   10                  15
   Tyr Ala Arg Pro Ser Ser Ile Lys Thr Phe Glu Glu Tyr Lys Lys Ala
               20                  25                  30
   Phe Asn Lys Ser Tyr Ala Thr Phe Glu Asp Glu Glu Ala Ala Arg Lys
           35                  40                  45
   Asn Phe Leu Glu Ser Val Lys Tyr Val Gln Ser Asn Gly Gly Ala Ile
       50                  55                  60
   Asn His Leu Ser Asp Leu Ser Leu Asp Glu Phe Lys Asn Arg Phe Leu
   65                  70                  75                  80
   Met Ser Ala Glu Ala Phe Glu His Leu Lys Thr Gln Phe Asp Leu Asn
                   85                  90                  95
   Ala Glu Ser Val Pro Ala Ser Val Asp Trp Arg Lys Lys Gly Ala Val
               100                 105                 110
   Thr Asp Val Lys Asp Gln Gly Gln Cys Gly Ser Cys Trp Ala Phe Ser
           115                 120                 125
   Thr Ile Val Ala Val Glu Gly Ile Asn Gln Ile Lys Thr Asn Lys Leu
       130                 135                 140
   Val Ser Leu Ser Glu Gln Glu Leu Val Asp Cys Asp Lys Glu Glu Asn
   145                 150                 155                 160
   Gln Gly Cys Asn Gly Gly Leu Met Glu Ser Ala Phe Glu Phe Ile Lys
                   165                 170                 175
   Gln Lys Gly Gly Ile Thr Thr Glu Ser Asn Tyr Pro Tyr Thr Ala Gln
               180                 185                 190
   Glu Gly Thr Cys Asp Glu Ser Lys Val Asn Asp Leu Ala Val Ser Ile
           195                 200                 205
   Asp Gly His Glu Asn Val Pro Val Asn Asp Glu Asn Ala Leu Leu Lys
       210                 215                 220
   Ala Val Ala Asn Gln Pro Val Ser Val Ala Ile Asp Ala Gly Gly Ser
   225                 230                 235                 240
   Asp Phe Gln Phe Tyr Ser Glu Gly Val Phe Thr Gly Asp Cys Asn Thr
                   245                 250                 255
   Asp Leu Asn His Gly Val Ala Ile Val Gly Tyr Gly Thr Thr Val Asp
               260                 265                 270
   Gly Thr Asn Tyr Trp Ile Val Arg Asn Ser Trp Gly Pro Glu Trp Gly
           275                 280                 285
   Glu Gln Gly Tyr Ile Arg Met Gln Arg Asn Ile Ser Lys Lys Glu Gly
       290                 295                 300
   Leu Cys Gly Ile Ala Met Met Ala Ser Tyr Pro Ile Lys Asn Ser Ser
   305                 310                 315                 320
   Asp Asn Pro Thr Gly Ser Leu Ser Ser Pro Lys Asp Glu Leu
                   325                 330
<210> 6
   <211> 320
   <212> PRT
   <213> Artificial Sequence
<220>
   <223> mutant
<400> 6
   Met Lys Ile Val Leu Ala Ile Ala Ser Leu Leu Ala Leu Ser Ala Val
   1               5                   10                  15
   Tyr Ala Arg Pro Ser Ser Ile Lys Thr Phe Glu Glu Tyr Lys Lys Ala
               20                  25                  30
   Phe Asn Lys Ser Tyr Ala Thr Phe Glu Asp Glu Glu Ala Ala Arg Lys
           35                  40                  45
   Asn Phe Leu Glu Ser Val Lys Tyr Val Gln Ser Asn Gly Gly Ala Ile
       50                  55                  60
   Asn His Leu Ser Asp Leu Ser Leu Asp Glu Phe Lys Asn Arg Phe Leu
   65                  70                  75                  80
   Met Ser Ala Glu Ala Phe Glu His Leu Lys Thr Gln Phe Asp Leu Asn
                   85                  90                  95
   Ala Lys Thr Asn Ala Cys Ser Ile Asn Gly Asn Ala Pro Ala Glu Ile
               100                 105                 110
   Asp Leu Arg Gln Met Arg Thr Val Thr Pro Ile Arg Met Gln Gly Gly
           115                 120                 125
   Cys Gly Ser Cys Trp Ala Phe Ser Gly Val Ala Ala Thr Glu Ser Ala
       130                 135                 140
   Tyr Leu Ala Tyr Arg Asn Gln Ser Leu Asp Leu Ala Glu Gln Glu Leu
   145                 150                 155                 160
   Val Asp Cys Ala Ser Gln His Gly Cys His Gly Asp Thr Ile Pro Arg
                   165                 170                 175
   Gly Ile Glu Tyr Ile Gln His Asn Gly Val Val Gln Glu Ser Tyr Tyr
               180                 185                 190
   Arg Tyr Val Ala Arg Glu Gln Ser Cys Arg Arg Pro Asn Ala Gln Arg
           195                 200                 205
   Phe Gly Ile Ser Asn Tyr Cys Gln Ile Tyr Pro Pro Asn Val Asn Lys
   210 215 220
   Ile Arg Glu Ala Leu Ala Gln Thr His Ser Ala Ile Ala Val Ile Ile
   225 230 235 240
   Gly Ile Lys Asp Leu Asp Ala Phe Arg His Tyr Asp Gly Arg Thr Ile
   245 250 255
   Ile Gln Arg Asp Asn Gly Tyr Gln Pro Asn Tyr His Ala Val Asn Ile
   260 265 270
   Val Gly Tyr Ser Asn Ala Gln Gly Val Asp Tyr Trp Ile Val Arg Asn
   275 280 285
   Ser Trp Asp Thr Asn Trp Gly Asp Asn Gly Tyr Gly Tyr Phe Ala Ala
   290 295 300
   Asn Ile Asp Leu Met Met Ile Glu Glu Tyr Pro Tyr Val Val Ile Leu
   305 310 315 320
<210> 7
   <211> 320
   <212> PRT
   <213> Artificial Sequence
<220>
   <223> mutant
<400> 7
   Met Lys Ile Val Leu Ala Ile Ala Ser Leu Leu Ala Leu Ser Ala Val
   1               5                   10                  15
   Tyr Ala Arg Pro Ser Ser Ile Lys Thr Phe Glu Glu Tyr Lys Lys Ala
               20                  25                  30
   Phe Gln Lys Ser Tyr Ala Thr Phe Glu Asp Glu Glu Ala Ala Arg Lys
           35                  40                  45
   Asn Phe Leu Glu Ser Val Lys Tyr Val Gln Ser Asn Gly Gly Ala Ile
       50                  55                  60
   Asn His Leu Ser Asp Leu Ser Leu Asp Glu Phe Lys Asn Arg Phe Leu
   65                  70                  75                  80
   Met Ser Ala Glu Ala Phe Glu His Leu Lys Thr Gln Phe Asp Leu Asn
                   85                  90                  95
   Ala Glu Thr Asn Ala Cys Ser Ile Asn Gly Asn Ala Pro Ala Glu Ile
               100                 105                 110
   Asp Leu Arg Gln Met Arg Thr Val Thr Pro Ile Arg Met Gln Gly Gly
   115 120 125
   Cys Gly Ser Cys Trp Ala Phe Ser Gly Val Ala Ala Thr Glu Ser Ala
   130 135 140
   Tyr Leu Ala Tyr Arg Asn Gln Ser Leu Asp Leu Ala Glu Gln Glu Leu
   145 150 155 160
   Val Asp Cys Ala Ser Gln His Gly Cys His Gly Asp Thr Ile Pro Arg
   165 170 175
   Gly Ile Glu Tyr Ile Gln His Asn Gly Val Val Gln Glu Ser Tyr Tyr
   180 185 190
   Arg Tyr Val Ala Arg Glu Gln Ser Cys Arg Arg Pro Asn Ala Gln Arg
   195 200 205
   Phe Gly Ile Ser Asn Tyr Cys Gln Ile Tyr Pro Pro Asn Val Asn Lys
   210 215 220
   Ile Arg Glu Ala Leu Ala Gln Thr His Ser Ala Ile Ala Val Ile Ile
   225 230 235 240
   Gly Ile Lys Asp Leu Asp Ala Phe Arg His Tyr Asp Gly Arg Thr Ile
   245 250 255
   Ile Gln Arg Asp Asn Gly Tyr Gln Pro Asn Tyr His Ala Val Asn Ile
   260 265 270
   Val Gly Tyr Ser Asn Ala Gln Gly Val Asp Tyr Trp Ile Val Arg Asn
   275 280 285
   Ser Trp Asp Thr Asn Trp Gly Asp Asn Gly Tyr Gly Tyr Phe Ala Ala
   290 295 300
   Asn Ile Asp Leu Met Met Ile Glu Glu Tyr Pro Tyr Val Val Ile Leu
   305 310 315 320
<210> 8
   <211> 80
   <212> PRT
   <213> Artificial Sequence
<220>
   <223> propeptide Der p1
<400> 8
   Arg Pro Ser Ser Ile Lys Thr Phe Glu Glu Tyr Lys Lys Ala Phe Asn
   1 5 10 15
   Lys Ser Tyr Ala Thr Phe Glu Asp Glu Glu Ala Ala Arg Lys Asn Phe
   20 25 30
   Leu Glu Ser Val Lys Tyr Val Gln Ser Asn Gly Gly Ala Ile Asn His
   35 40 45
   Leu Ser Asp Leu Ser Leu Asp Glu Phe Lys Asn Arg Phe Leu Met Ser
   50 55 60
   Ala Glu Ala Phe Glu His Leu Lys Thr Gln Phe Asp Leu Asn Ala Glu
   65 70 75 80
<210> 9
   <211> 80
   <212> PRT
   <213> Artificial Sequence
<220>
   <223> propeptide modifié
<400> 9
   Arg Pro Ser Ser Ile Lys Thr Phe Glu Glu Tyr Lys Lys Ala Phe Asn
   1 5 10 15
   Lys Ser Tyr Ala Thr Phe Glu Asp Glu Glu Ala Ala Arg Lys Asn Phe
   20 25 30
   Leu Glu Ser Val Lys Tyr Val Gln Ser Asn Gly Gly Ala Ile Asn His
   35 40 45
   Leu Ser Asp Leu Ser Leu Asp Glu Phe Lys Asn Arg Phe Leu Met Ser
   50 55 60
   Ala Glu Ala Phe Glu His Leu Lys Thr Gln Phe Asp Leu Asn Ala Lys
   65 70 75 80
<210> 10
   <211> 254
   <212> PRT
   <213> Artificial Sequence
<220>
   <223> AMB A11 modifiée en C-terminal
<400> 10
   Gly Ser Ala Pro Gly Ser Ile Asp Thr Asp Pro Asn Lys Asp Phe Ile
   1 5 10 15
   Tyr Ala Asn Val Thr Lys Ile Pro Asp Lys Val Asp Trp Arg Glu Lys
               20                  25                  30
   Asn Ala Val Thr Asp Val Lys Gly Gln Gly Gly Cys Gly Ser Cys Trp
           35                  40                  45
   Ala Phe Ala Ala Val Val Ala Leu Glu Gly Ile Asn Ala Ile Arg Thr
       50                  55                  60
   Gly Lys Leu Val Lys Phe Ser Glu Gln Gln Leu Val Asp Cys Asp Met
   65                  70                  75                  80
   Thr Asn Ala Gly Cys Asp Gly Gly Leu Met Glu Pro Ala Phe Thr Tyr
                   85                  90                  95
   Val Ile Lys His Gly Gly Ile Ala Pro Glu Ala Ser Tyr Pro Tyr Val
               100                 105                 110
   Gly Lys Arg Glu Thr Cys Asp Lys Ala Lys Ile Lys Asp Val Leu Lys
           115                 120                 125
   Ile Asp Gly Arg Gln Asn Val Pro Gly Leu Asp Glu Glu Ala Leu Arg
       130                 135                 140
   Lys Ala Val Ala His Gln Pro Val Ala Thr Gly Ile Gln Leu Ser Gly
   145                 150                 155                 160
   His Gly Leu Gln Phe Tyr Ser Glu Gly Val Tyr Thr Gly Asp Cys Gly
                   165                 170                 175
   Thr Glu Pro Asn His Gly Val Gly Ile Val Gly Tyr Gly Glu Asn Glu
               180                 185                 190
   Lys Gly Ile Lys Phe Trp Thr Val Lys Asn Ser Trp Gly Pro Thr Trp
           195                 200                 205
   Gly Glu Lys Gly Tyr Ile His Leu Gln Arg Gly Ala Arg Lys Glu Gly
       210                 215                 220
   Leu Cys Gly Val Ala Met His Ser Ser Phe Pro Ile Met Asn Asp Pro
   225                 230                 235                 240
   Asn Pro Pro Lys Asp Asp Pro Asn Gly Pro Lys Asp Glu Leu
                   245                 250
<210> 11
   <211> 278
   <212> PRT
   <213> Artificial Sequence
<220>
   <223> protéine de fusion
<400> 11
   Gly Ser Ala Pro Gly Ser Ile Asp Thr Asp Pro Asn Lys Asp Phe Ile
   1               5                   10                  15
   Tyr Ala Asn Val Thr Lys Ile Pro Asp Lys Val Asp Trp Arg Glu Lys
               20                  25                  30
   Asn Ala Val Thr Asp Val Lys Gly Gln Gly Gly Cys Gly Ser Cys Trp
           35                  40                  45
   Ala Phe Ala Ala Val Val Ala Leu Glu Gly Ile Asn Ala Ile Arg Thr
       50                  55                  60
   Gly Lys Leu Val Lys Phe Ser Glu Gln Gln Leu Val Asp Cys Asp Met
   65                  70                  75                  80
   Thr Asn Ala Gly Cys Asp Gly Gly Leu Met Glu Pro Ala Phe Thr Tyr
                   85                  90                  95
   Val Ile Lys His Gly Gly Ile Ala Pro Glu Ala Ser Tyr Pro Tyr Val
               100                 105                 110
   Gly Lys Arg Glu Thr Cys Asp Lys Ala Lys Ile Lys Asp Val Leu Lys
           115                 120                 125
   Ile Asp Gly Arg Gln Asn Val Pro Gly Leu Asp Glu Glu Ala Leu Arg
       130                 135                 140
   Lys Ala Val Ala His Gln Pro Val Ala Thr Gly Ile Gln Leu Ser Gly
   145                 150                 155                 160
   His Gly Leu Gln Phe Tyr Ser Glu Gly Val Tyr Thr Gly Asp Cys Gly
                   165                 170                 175
   Thr Glu Pro Asn His Gly Val Gly Ile Val Gly Tyr Gly Glu Asn Glu
               180                 185                 190
   Lys Gly Ile Lys Phe Trp Thr Val Lys Asn Ser Trp Gly Pro Thr Trp
           195                 200                 205
   Gly Glu Lys Gly Tyr Ile His Leu Gln Arg Gly Ala Arg Lys Glu Gly
       210                 215                 220
   Leu Cys Gly Val Ala Met His Ser Ser Phe Pro Ile Met Asn Asp Pro
   225                 230                 235                 240
   Asn Pro Pro Lys Asp Asp Pro Asn Gly Pro Lys Asp Asp Pro Asp Ala
                   245                 250                 255
   Pro Lys Asp Pro Lys Phe Lys Thr Thr Gln Arg Leu Gln Gly Ile Arg
               260                 265                 270
   Thr Lys Leu Leu Glu Leu
           275
<210> 12
   <211> 254
   <212> PRT
   <213> Artificial Sequence
<220>
   <223> protéine de fusion
<400> 12
   Gly Ser Ala Pro Gly Ser Ile Asp Thr Asp Pro Asn Lys Asp Phe Ile
   1               5                   10                  15
   Tyr Ala Asn Val Thr Lys Ile Pro Asp Lys Val Asp Trp Arg Glu Lys
               20                  25                  30
   Asn Ala Val Thr Asp Val Lys Gly Gln Gly Gly Cys Gly Ser Cys Trp
           35                  40                  45
   Ala Phe Ala Ala Val Val Ala Leu Glu Gly Ile Asn Ala Ile Arg Thr
       50                  55                  60
   Gly Lys Leu Val Lys Phe Ser Glu Gln Gln Leu Val Asp Cys Asp Met
   65                  70                  75                  80
   Thr Asn Ala Gly Cys Asp Gly Gly Leu Met Glu Pro Ala Phe Thr Tyr
                   85                  90                  95
   Val Ile Lys His Gly Gly Ile Ala Pro Glu Ala Ser Tyr Pro Tyr Val
               100                 105                 110
   Gly Lys Arg Glu Thr Cys Asp Lys Ala Lys Ile Lys Asp Val Leu Lys
           115                 120                 125
   Ile Asp Gly Arg Gln Asn Val Pro Gly Leu Asp Glu Glu Ala Leu Arg
       130                 135                 140
   Lys Ala Val Ala His Gln Pro Val Ala Thr Gly Ile Gln Leu Ser Gly
   145                 150                 155                 160
   His Gly Leu Gln Phe Tyr Ser Glu Gly Val Tyr Thr Gly Asp Cys Gly
                   165                 170                 175
   Thr Glu Pro Asn His Gly Val Gly Ile Val Gly Tyr Gly Glu Asn Glu
               180                 185                 190
   Lys Gly Ile Lys Phe Trp Thr Val Lys Asn Ser Trp Gly Pro Thr Trp
           195                 200                 205
   Gly Glu Lys Gly Tyr Ile His Leu Gln Arg Gly Ala Arg Lys Glu Gly
       210                 215                 220
   Leu Cys Gly Val Ala Met His Ser Ser Phe Pro Ile Met Asn Asp Pro
   225                 230                 235                 240
   Asn Pro Pro Lys Asp Asp Pro Asn Gly Pro Lys Asp Glu Leu
                   245                 250
<210> 13
   <211> 106
   <212> PRT
   <213> Artificial Sequence
<220>
   <223> propeptide naturel de SH-EP
<400> 13
   Asn Ser Phe Asp Phe His Glu Lys Asp Leu Glu Ser Glu Glu Ser Leu
   1               5                   10                  15
   Trp Asp Leu Tyr Glu Arg Trp Arg Ser His His Thr Val Ser Arg Ser
               20                  25                  30
   Leu Gly Glu Lys His Lys Arg Phe Asn Val Phe Lys Ala Asn Val Met
           35                  40                  45
   His Val His Asn Thr Asn Lys Met Asp Lys Pro Tyr Lys Leu Lys Leu
       50                  55                  60
   Asn Lys Phe Ala Asp Met Thr Asn His Glu Phe Arg Ser Thr Tyr Ala
   65                  70                  75                  80
   Gly Ser Lys Val Asn His His Lys Met Phe Arg Gly Ser Gln His Gly
                   85                  90                  95
   Ser Gly Thr Phe Met Tyr Glu Lys Val Gly
   100 105
<210> 14
   <211> 362
   <212> PRT
   <213> Artificial Sequence
<220>
   <223> Préproprotéine AmoA11 modifiée
<400> 14
   Met Glu Ile Asn Lys Leu Val Cys Phe Ser Phe Ser Leu Val Leu Ile
   1 5 10 15
   Leu Gly Leu Val Glu Ser Phe His Tyr His Glu Arg Glu Leu Glu Ser
   20 25 30
   Glu Glu Gly Phe Met Gly Met Tyr Asp Arg Trp Arg Glu Gln His Asn
   35 40 45
   Ile Glu Met Arg Ser Pro Glu Arg Phe Asn Val Phe Lys Tyr Asn Val
   50 55 60
   Arg Arg Ile His Glu Ser Asn Lys Met Asp Lys Pro Tyr Lys Leu Lys
   65 70 75 80
   Val Asn Glu Phe Ala Asp Met Thr Asn Leu Glu Phe Val Asn Thr Tyr
   85 90 95
   Ala Asn Ser Lys Ile Ser His Phe Gln Ala Leu Arg Gly Ser Ala Pro
   100 105 110
   Gly Ser Ile Asp Thr Asp Pro Asn Lys Asp Phe Ile Tyr Ala Asn Val
   115 120 125
   Thr Lys Ile Pro Asp Lys Val Asp Trp Arg Glu Lys Asn Ala Val Thr
   130 135 140
   Asp Val Lys Gly Gln Gly Gly Cys Gly Ser Cys Trp Ala Phe Ala Ala
   145 150 155 160
   Val Val Ala Leu Glu Gly Ile Asn Ala Ile Arg Thr Gly Lys Leu Val
   165 170 175
   Lys Phe Ser Glu Gln Gln Leu Val Asp Cys Asp Met Thr Asn Ala Gly
   180 185 190
   Cys Asp Gly Gly Leu Met Glu Pro Ala Phe Thr Tyr Val Ile Lys His
               195                 200                 205
   Gly Gly Ile Ala Pro Glu Ala Ser Tyr Pro Tyr Val Gly Lys Arg Glu
       210                 215                 220
   Thr Cys Asp Lys Ala Lys Ile Lys Asp Val Leu Lys Ile Asp Gly Arg
   225                 230                 235                 240
   Gln Asn Val Pro Gly Leu Asp Glu Glu Ala Leu Arg Lys Ala Val Ala
                   245                 250                 255
   His Gln Pro Val Ala Thr Gly Ile Gln Leu Ser Gly His Gly Leu Gln
               260                 265                 270
   Phe Tyr Ser Glu Gly Val Tyr Thr Gly Asp Cys Gly Thr Glu Pro Asn
           275                 280                 285
   His Gly Val Gly Ile Val Gly Tyr Gly Glu Asn Glu Lys Gly Ile Lys
       290                 295                 300
   Phe Trp Thr Val Lys Asn Ser Trp Gly Pro Thr Trp Gly Glu Lys Gly
   305                 310                 315                 320
   Tyr Ile His Leu Gln Arg Gly Ala Arg Lys Glu Gly Leu Cys Gly Val
                   325                 330                 335
   Ala Met His Ser Ser Phe Pro Ile Met Asn Asp Pro Asn Pro Pro Lys
               340                 345                 350
   Asp Asp Pro Asn Gly Pro Lys Asp Glu Leu
           355                 360
<210> 15
   <211> 404
   <212> PRT
   <213> Artificial Sequence
<220>
   <223> Préproprotéine de fusion
<400> 15
   Met Ala Met Lys Lys Leu Leu Trp Val Val Leu Ser Leu Ser Leu Val
   1               5                   10                  15
   Leu Gly Val Ala Asn Ser Phe Asp Phe His Glu Lys Asp Leu Glu Ser
               20                  25                  30
   Glu Glu Ser Leu Trp Asp Leu Tyr Glu Arg Trp Arg Ser His His Thr
           35                  40                  45
   Val Ser Arg Ser Leu Gly Glu Lys His Lys Arg Phe Asn Val Phe Lys
       50                  55                  60
   Ala Asn Val Met His Val His Asn Thr Asn Lys Met Asp Lys Pro Tyr
   65                  70                  75                  80
   Lys Leu Lys Leu Asn Lys Phe Ala Asp Met Thr Asn His Glu Phe Arg
                   85                  90                  95
   Ser Thr Tyr Ala Gly Ser Lys Val Asn His His Lys Met Phe Arg Gly
               100                 105                 110
   Ser Gln His Gly Ser Gly Thr Phe Met Tyr Glu Lys Val Gly Gly Ser
           115                 120                 125
   Ala Pro Gly Ser Ile Asp Thr Asp Pro Asn Lys Asp Phe Ile Tyr Ala
       130                 135                 140
   Asn Val Thr Lys Ile Pro Asp Lys Val Asp Trp Arg Glu Lys Asn Ala
   145                 150                 155                 160
   Val Thr Asp Val Lys Gly Gln Gly Gly Cys Gly Ser Cys Trp Ala Phe
                   165                 170                 175
   Ala Ala Val Val Ala Leu Glu Gly Ile Asn Ala Ile Arg Thr Gly Lys
               180                 185                 190
   Leu Val Lys Phe Ser Glu Gln Gln Leu Val Asp Cys Asp Met Thr Asn
           195                 200                 205
   Ala Gly Cys Asp Gly Gly Leu Met Glu Pro Ala Phe Thr Tyr Val Ile
       210                 215                 220
   Lys His Gly Gly Ile Ala Pro Glu Ala Ser Tyr Pro Tyr Val Gly Lys
   225                 230                 235                 240
   Arg Glu Thr Cys Asp Lys Ala Lys Ile Lys Asp Val Leu Lys Ile Asp
                   245                 250                 255
   Gly Arg Gln Asn Val Pro Gly Leu Asp Glu Glu Ala Leu Arg Lys Ala
               260                 265                 270
   Val Ala His Gln Pro Val Ala Thr Gly Ile Gln Leu Ser Gly His Gly
           275                 280                 285
   Leu Gln Phe Tyr Ser Glu Gly Val Tyr Thr Gly Asp Cys Gly Thr Glu
       290                 295                 300
   Pro Asn His Gly Val Gly Ile Val Gly Tyr Gly Glu Asn Glu Lys Gly
   305                 310                 315                 320
   Ile Lys Phe Trp Thr Val Lys Asn Ser Trp Gly Pro Thr Trp Gly Glu
                   325                 330                 335
   Lys Gly Tyr Ile His Leu Gln Arg Gly Ala Arg Lys Glu Gly Leu Cys
               340                 345                 350
   Gly Val Ala Met His Ser Ser Phe Pro Ile Met Asn Asp Pro Asn Pro
           355                 360                 365
   Pro Lys Asp Asp Pro Asn Gly Pro Lys Asp Asp Pro Asp Ala Pro Lys
       370                 375                 380
   Asp Pro Lys Phe Lys Thr Thr Gln Arg Leu Gln Gly Ile Arg Thr Lys
   385                 390                 395                 400
   Leu Leu Glu Leu
<210> 16
   <211> 380
   <212> PRT
   <213> Artificial Sequence
<220>
   <223> Préproprotéine de fusion
<400> 16
   Met Ala Met Lys Lys Leu Leu Trp Val Val Leu Ser Leu Ser Leu Val
   1               5                   10                  15
   Leu Gly Val Ala Asn Ser Phe Asp Phe His Glu Lys Asp Leu Glu Ser
               20                  25                  30
   Glu Glu Ser Leu Trp Asp Leu Tyr Glu Arg Trp Arg Ser His His Thr
           35                  40                  45
   Val Ser Arg Ser Leu Gly Glu Lys His Lys Arg Phe Asn Val Phe Lys
       50                  55                  60
   Ala Asn Val Met His Val His Asn Thr Asn Lys Met Asp Lys Pro Tyr
   65                  70                  75                  80
   Lys Leu Lys Leu Asn Lys Phe Ala Asp Met Thr Asn His Glu Phe Arg
                   85                  90                  95
   Ser Thr Tyr Ala Gly Ser Lys Val Asn His His Lys Met Phe Arg Gly
               100                 105                 110
   Ser Gln His Gly Ser Gly Thr Phe Met Tyr Glu Lys Val Gly Gly Ser
           115                 120                 125
   Ala Pro Gly Ser Ile Asp Thr Asp Pro Asn Lys Asp Phe Ile Tyr Ala
       130                 135                 140
   Asn Val Thr Lys Ile Pro Asp Lys Val Asp Trp Arg Glu Lys Asn Ala
   145                 150                 155                 160
   Val Thr Asp Val Lys Gly Gln Gly Gly Cys Gly Ser Cys Trp Ala Phe
                   165                 170                 175
   Ala Ala Val Val Ala Leu Glu Gly Ile Asn Ala Ile Arg Thr Gly Lys
               180                 185                 190
   Leu Val Lys Phe Ser Glu Gln Gln Leu Val Asp Cys Asp Met Thr Asn
           195                 200                 205
   Ala Gly Cys Asp Gly Gly Leu Met Glu Pro Ala Phe Thr Tyr Val Ile
       210                 215                 220
   Lys His Gly Gly Ile Ala Pro Glu Ala Ser Tyr Pro Tyr Val Gly Lys
   225                 230                 235                 240
   Arg Glu Thr Cys Asp Lys Ala Lys Ile Lys Asp Val Leu Lys Ile Asp
                   245                 250                 255
   Gly Arg Gln Asn Val Pro Gly Leu Asp Glu Glu Ala Leu Arg Lys Ala
               260                 265                 270
   Val Ala His Gln Pro Val Ala Thr Gly Ile Gln Leu Ser Gly His Gly
           275                 280                 285
   Leu Gln Phe Tyr Ser Glu Gly Val Tyr Thr Gly Asp Cys Gly Thr Glu
       290                 295                 300
   Pro Asn His Gly Val Gly Ile Val Gly Tyr Gly Glu Asn Glu Lys Gly
   305                 310                 315                 320
   Ile Lys Phe Trp Thr Val Lys Asn Ser Trp Gly Pro Thr Trp Gly Glu
                   325                 330                 335
   Lys Gly Tyr Ile His Leu Gln Arg Gly Ala Arg Lys Glu Gly Leu Cys
                   340                 345                 350
   Gly Val Ala Met His Ser Ser Phe Pro Ile Met Asn Asp Pro Asn Pro
           355                 360                 365
   Pro Lys Asp Asp Pro Asn Gly Pro Lys Asp Glu Leu
       370                 375                 380